# EUROPEAN PATENT APPLICATION

(11) **EP 4 803 002 A1**
(43) Date of publication of application: **09.09.2026**
(21) Application number: 24904176.5
(22) Date of filing: 05.12.2024
(51) Int. Cl.: A61B 5/1495, A61B 5/145, A61B 5/1455, A61B 5/00

(54) **WEARABLE ELECTRONIC DEVICE FOR ACQUIRING CALIBRATION DATA RELATED TO BLOOD GLUCOSE VALUES, OPERATING METHOD THEREOF, AND STORAGE MEDIUM**

(30) Priority: 11.12.2023 KR 20230179031; 07.02.2024 KR 20240018618
(71) Applicant: Samsung Electronics Co., Ltd., Suwon-si, Gyeonggi-do 16677 (KR)
(72) Inventor: SEO, Hyejung, Suwon-si Gyeonggi-do 16677 (KR); PARK, Jeongmin, Suwon-si Gyeonggi-do 16677 (KR)
(74) Representative: Appleyard Lees IP LLP
(86) International application number: PCT/KR2024/019861
(87) International publication number: WO 2025/127611

(57) **Abstract**

According to one embodiment, a wearable electronic device comprises a first sensor, at least one second sensor, a memory, a display, a communication circuit and at least one processor, wherein the memory can store instructions that, when executed by the at least one processor, cause the wearable electronic device to: identify, on the basis of a first sensing value identified through the at least one second sensor, a first time point so as to measure first blood glucose data of a user wearing the wearable electronic device; use an external device at the first time point so as to display, through the display, first guide information for allowing the blood glucose of the user to be measured; measure, through the first sensor, a second sensing value indicating a first biometric signal of the user at the first time point; acquire, through the communication circuit, information indicating a first blood glucose value of the user measured at the first time point by the external device; and acquire, on the basis of the first blood glucose value and a second sensing value indicating the first biometric signal, calibration data for determining a blood glucose value of the user on the basis of sensing values indicating biometric signals of the user to be measured using the first sensor.

## Description

### [Technical Field]

Embodiments of the disclosure relate to a wearable electronic device obtaining calibration data related to a blood sugar value, an operating method thereof, and a storage medium.

### [Background Art]

Nowadays, lifestyle diseases have increased due to enhanced living environments, and thus users have become more interested in health than before. In particular, the number of diabetes patients is increasing. A diabetes patient should periodically check a blood sugar value to track his or her condition at any time and take an appropriate response thereto.

Currently, a blood sampling glucometer is one of medical devices measuring blood sugar. The blood sampling glucometer is a device that penetrates a needle beneath a user's skin to collect blood and measures blood sugar through the collected blood. However, such a method may cause pain to the patient during blood sampling. Accordingly, a problem has arisen that the patient avoids or evades blood sugar measurement, and thus there has been a limitation that blood sugar management is not properly performed because the patient may not frequently check his or her blood sugar level.

The information may be provided as background technology (related art) for the purpose of helping to understand the disclosure. No claim or determination is made as to whether any of the foregoing is applicable as background art in relation to the disclosure.

### [Detailed Description of the Invention]

### [Technical Solution]

According to an embodiment, a wearable electronic device may include a first sensor, at least one second sensor, memory, a display, communication circuitry, and at least one processor.

According to an embodiment, the memory may store instructions that, when executed by the at least one processor, cause the wearable electronic device to identify a first time point to measure first blood sugar data of a user wearing the wearable electronic device based on a first sensing value identified through the at least one second sensor.

According to an embodiment, the memory may store instructions that, when executed by the at least one processor, cause the wearable electronic device to display, through the display, first guide information for measuring a blood sugar of the user at the first time point using an external device.

According to an embodiment, the memory may store instructions that, when executed by the at least one processor, cause the wearable electronic device to measure, through the first sensor, a second sensing value indicating a first biometric signal of the user at the first time point.

According to an embodiment, the memory may store instructions that, when executed by the at least one processor, cause the wearable electronic device to obtain, through the communication circuitry, information indicating a first blood sugar value of the user measured by the external device at the first time point.

According to an embodiment, the memory may store instructions that, when executed by the at least one processor, cause the wearable electronic device to obtain the calibration data for determining a blood sugar value of the user based on a sensing value indicating a biometric signal value of the user measured using the first sensor, based on the second sensing value indicating the first biometric signal and the first blood sugar value.

According to an embodiment, a method of operating a wearable electronic device may include an operation of identifying a first time point to measure first blood sugar data of a user wearing the wearable electronic device to obtain calibration data based on a first sensing value identified through at least one second sensor included in the wearable electronic device.

According to an embodiment, a method of operating a wearable electronic device may include an operation of displaying, through a display included in the wearable electronic device, first guide information for measuring a blood sugar of the user at the first time point using an external device.

According to an embodiment, a method of operating a wearable electronic device may include an operation of measuring, through a first sensor included in the wearable electronic device, a second sensing value indicating a first biometric signal of the user at the first time point.

According to an embodiment, a method of operating a wearable electronic device may include an operation of obtaining, through communication circuitry included in the wearable electronic device, information indicating a first blood sugar value of the user measured by the external device at the first time point.

According to an embodiment, a method of operating a wearable electronic device may include an operation of obtaining the calibration data for determining a blood sugar value of the user based on a sensing value indicating a biometric signal of the user to be measured using the first sensor, based on the second sensing value indicating the first biometric signal and the first blood sugar value.

According to an embodiment, in a storage medium storing computer-readable instructions, the instructions, when executed by at least one processor of a wearable electronic device, cause the wearable electronic device to perform at least one operation. The at least one operation may include an operation of identifying a first time point to measure first blood sugar data of a user wearing the wearable electronic device to obtain calibration data based on a first sensing value identified through at least one second sensor included in the wearable electronic device.

According to an embodiment, in a storage medium storing computer-readable instructions, the instructions, when executed by at least one processor of a wearable electronic device, cause the wearable electronic device to perform at least one operation. The at least one operation may include an operation of displaying, through a display included in the wearable electronic device, first guide information for measuring a blood sugar of the user at the first time point using an external device.

According to an embodiment, in a storage medium storing computer-readable instructions, the instructions, when executed by at least one processor of a wearable electronic device, cause the wearable electronic device to perform at least one operation. The at least one operation may include an operation of measuring, through a first sensor included in the wearable electronic device, a second sensing value indicating a first biometric signal of the user at the first time point.

According to an embodiment, in a storage medium storing computer-readable instructions, the instructions, when executed by at least one processor of a wearable electronic device, cause the wearable electronic device to perform at least one operation. The at least one operation may include an operation of obtaining, through communication circuitry included in the wearable electronic device, information indicating a first blood sugar value of the user measured by the external device at the first time point.

According to an embodiment, in a storage medium storing computer-readable instructions, the instructions, when executed by at least one processor of a wearable electronic device, cause the wearable electronic device to perform at least one operation. The at least one operation may include an operation of obtaining the calibration data for determining a blood sugar value of the user based on a sensing value indicating a biometric signal of the user to be measured using the first sensor, based on the second sensing value indicating the first biometric signal and the first blood sugar value.

### [Brief Description of Drawings]

FIG. 1 is a view illustrating an electronic device in a network environment according to an embodiment.
FIG. 2 is a schematic block diagram illustrating a system including a wearable electronic device and an external device according to an embodiment.
FIG. 3 is a flowchart illustrating an operation of a wearable electronic device obtaining calibration data according to an embodiment.
FIG. 4 is a flowchart illustrating an operation of a wearable electronic device determining a time point to measure blood sugar data based on a meal event according to an embodiment.
FIG. 5 is a flowchart illustrating an operation of a wearable electronic device determining a time point to measure blood sugar data based on a meal event according to an embodiment.
FIG. 6 is a flowchart illustrating an operation of a wearable electronic device determining a time point to measure blood sugar data based on an exercise event according to an embodiment.
FIG. 7 is a flowchart illustrating an operation of a wearable electronic device determining a time point to measure blood sugar data based on a wake-up event according to an embodiment.
FIG. 8 is a flowchart illustrating an operation of a wearable electronic device obtaining calibration data according to an embodiment.
FIG. 9A is a flowchart illustrating an operation of a wearable electronic device determining a blood sugar value based on a sensing value measured through a first sensor using calibration data according to an embodiment.
FIG. 9B is a flowchart illustrating an operation of a wearable electronic device updating calibration data according to an embodiment.
FIG. 10 is a view illustrating information on a blood sugar value measured by an external device according to an embodiment.
FIG. 11 is a view illustrating an operation of a wearable electronic device obtaining calibration data according to an embodiment.
FIG. 12 is a view illustrating information on a blood sugar value measured by an external device implemented as a continuous glucose monitoring system according to an embodiment.
FIG. 13 is a view illustrating a screen displayed through a display when a wearable electronic device executes a function of obtaining calibration data according to an embodiment.
FIG. 14 is a view illustrating a screen displayed through a display when a wearable electronic device identifies a wake-up event according to an embodiment.
FIG. 15 is a view illustrating a screen displayed through a display when a wearable electronic device identifies a meal event according to an embodiment.
FIG. 16 is a view illustrating a screen displayed through a display when a wearable electronic device identifies an exercise event according to an embodiment.
FIG. 17 is a view illustrating a screen displayed through a display when a wearable electronic device identifies that additional blood sugar data is needed according to an embodiment.
FIG. 18 is a view illustrating a screen displayed through a display when a wearable electronic device terminates a function of obtaining calibration data according to an embodiment.
FIG. 19 is a view illustrating a screen on which a wearable electronic device displays calibration data according to an embodiment.

### [Mode for Carrying out the Invention]

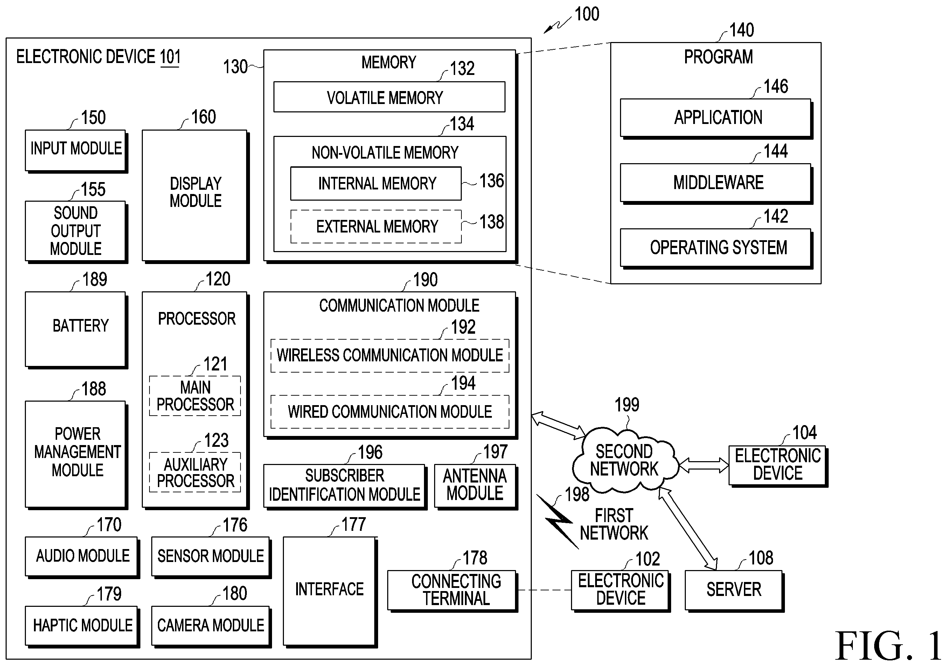

FIG. 1 is a block diagram illustrating an electronic device 101 in a network environment 100, according to various embodiments. Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., the program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operation state (e.g., power or temperature) of the electronic device 101 or an external environmental state (e.g., the user's state), and then generate an electrical signal or data value corresponding to the detected state. According to an embodiment, the sensor module 176 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wiredly) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wiredly) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to an embodiment, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, instructions or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

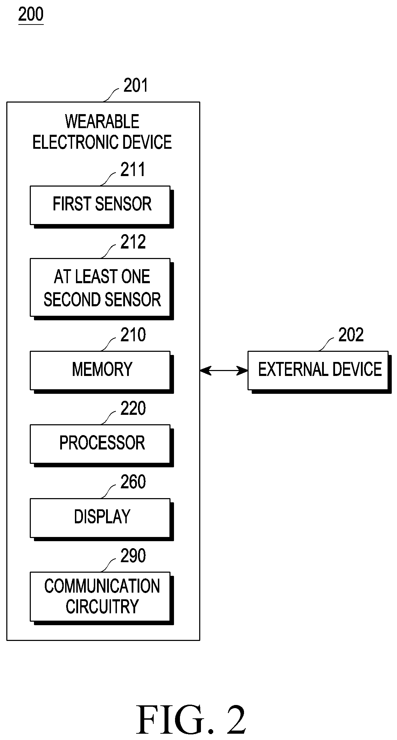

FIG. 2 is a schematic block diagram illustrating a system including a wearable electronic device and an external device according to an embodiment.

Referring to FIG. 2, a system 200 may include a wearable electronic device 201 and an external device 202.

According to an embodiment, the external device 202 may include a device measuring a blood sugar of a user in an invasive manner. For example, the external device 202 may be implemented as a self-monitoring blood glucometer (SMBG) or a continuous glucose monitoring system (CGMS) device. According to an embodiment, the wearable electronic device 201 may be implemented as a wearable electronic device wearable on a body (e.g., wrist) of the user. According to an embodiment, the wearable electronic device 201 may include a sensor (e.g., the first sensor 211) measuring a sensing value indicating a biometric signal of the user in a non-invasive manner.

According to an embodiment, the external device 202 may collect blood from a skin of the user to obtain a blood sugar value. According to an embodiment, the wearable electronic device 201 may radiate light onto a skin of the user using the first sensor 211, and measure light reflected from the skin to obtain a blood sugar value. According to an embodiment, a blood sugar value based on a sensing value measured by the first sensor 211 of the wearable electronic device 201 may be less accurate than a blood sugar value measured by the external device 202 because an amount of light reflected from the skin may vary according to a skin color of the user. A method of measuring a blood sugar of the user in an invasive manner using the external device 202 may be more accurate than a method of measuring a blood sugar of the user in a non-invasive manner, but may cause pain to the user. To solve such a problem, according to an embodiment, the wearable electronic device 201 may obtain calibration data for estimating a blood sugar value based on a sensing value indicating a biometric signal of the user obtained through the first sensor 211. The calibration data may be obtained based on a blood sugar value obtained from the external device 202 and a sensing value indicating a biometric signal measured through the first sensor 211 included in the wearable electronic device 201. According to an embodiment, the wearable electronic device 201 may determine a blood sugar value corresponding to a sensing value obtained through the first sensor 211 as a blood sugar value of the user using the calibration data. Accordingly, the wearable electronic device 201 may provide an accurate blood sugar value to the user.

According to an embodiment, the wearable electronic device 201 may include a first sensor 211 (e.g., the sensor module 176 of FIG. 1), at least one second sensor 212 (e.g., the sensor module 176 of FIG. 1), memory 210 (e.g., the memory 130 of FIG. 1), a processor 220 (e.g., the processor 120 of FIG. 1), a display 260 (e.g., the display module 160 of FIG. 1), and communication circuitry 290 (e.g., the communication module 190 of FIG. 1). According to an embodiment, the wearable electronic device 201 may be implemented to be identical or similar to the electronic device 101 of FIG. 1. However, this is merely an example, and the wearable electronic device 201 may be implemented as various devices.

According to an embodiment, the processor 220 may control the overall operation of the wearable electronic device 201. According to an embodiment, the processor 220 may be implemented identically or similarly to the processor 120 of FIG. 1.

According to an embodiment, the memory 210 may store instructions causing operations of the wearable electronic device 201 to be performed.

According to an embodiment, the first sensor 211 may include a sensor measuring a sensing value indicating a biometric signal of a user wearing the wearable electronic device 201. According to an embodiment, the first sensor 211 may include an optical sensor. For example, the processor 220 may radiate light output from a light emitting unit included in the first sensor 211 onto a body of the user, and obtain light reflected from the body of the user through a light receiving unit included in the first sensor 211. The processor 220 may measure a sensing value indicating a biometric signal of the user using the obtained reflected light. According to an embodiment, the first sensor 211 may include an electrode sensor. For example, the processor 220 may measure a sensing value indicating a biometric signal of the user based on a current value generated by applying a voltage to the first sensor 211. However, this is merely an example, and a sensor measuring a sensing value indicating a biometric signal of the user may be implemented as various sensors.

According to an embodiment, the at least one second sensor 212 may include an acceleration sensor, a proximity sensor, or a gyro sensor. According to an embodiment, the at least one second sensor 212 may include a photoplethysmogram (PPG) sensor. However, this is merely an example, and the at least one second sensor 212 may be implemented as various sensors.

According to an embodiment, the processor 220 may execute a function of obtaining calibration data. For example, the processor 220 may execute an application of the wearable electronic device 201 capable of executing a function of obtaining calibration data. According to an embodiment, the calibration data may include a calibration curve indicating a relationship between at least one blood sugar value of the user measured by the external device 202 and at least one sensing value indicating at least one biometric signal of the user measured through the first sensor 211. For example, the calibration curve may include a function formula between at least one blood sugar value of the user measured by the external device 202 and at least one sensing value indicating at least one biometric signal value of the user measured through the first sensor 211. The calibration curve may be implemented as an nth order (n is a natural number equal to or greater than 1) function.

Hereinafter, an operation in which the processor 220 according to an embodiment obtains calibration data is described.

According to an embodiment, the processor 220 may obtain a sensing value through the at least one second sensor 212. For example, the processor 220 may obtain a sensing value indicating at least one of a movement, a heart rate, an oxygen saturation, a stress, or a blood pressure of the user through the at least one second sensor 212.

According to an embodiment, the processor 220 may determine a time point to measure blood sugar data of a user wearing the wearable electronic device 201 based on the sensing value. For example, the blood sugar data may include a blood sugar value measured by the first sensor 211 at the determined time point and a blood sugar value measured by the external device 202. For example, the processor 220 may identify whether the user is in a stable state based on the sensing value. When it is identified that the user is in the stable state, the processor 220 may determine a time point to measure blood sugar data of the user wearing the wearable electronic device 201 based on the sensing value.

According to an embodiment, the processor 220 may determine whether at least one of a wake-up event indicating that the user has woken up, a meal event indicating that the user is eating, or an exercise event indicating that the user is exercising is identified based on the sensing value.

According to an embodiment, the processor 220 may determine a time point to measure blood sugar data based on at least one of the wake-up event, the meal event, or the exercise event. For example, the time point to measure blood sugar data may include a time point to measure a maximum blood sugar value, a minimum blood sugar value, or an intermediate blood sugar value of the user.

According to an embodiment, when the sensing value is included in a specified range corresponding to the wake-up event, the processor 220 may identify that the wake-up event occurs. According to an embodiment, the processor 220 may determine the time point where the wake-up event is identified as a time point to measure blood sugar data of the user.

According to an embodiment, when the sensing value is included in a specified range corresponding to the meal event, the processor 220 may identify that the meal event occurs. For example, the processor 220 may identify the meal event based on a sensing value indicating a movement of the user. For example, the processor 220 may identify the meal event by analyzing a sensing value indicating a movement of the user using an artificial intelligence model stored in the memory 210. The artificial intelligence model may include a temporal convolution net (TCN), a decision tree (DT), or a k-nearest neighbor (KNN). For example, the processor 220 may also identify the meal event based on a voice signal obtained through a microphone included in the wearable electronic device 201. In this case, the processor 220 may also identify the meal event by further considering a voice signal obtained from an external electronic device wearable on an ear of the user.

According to an embodiment, the processor 220 may determine a time point where a specified first time elapses from a time point where the meal event is identified as a time point to measure blood sugar data of the user. The specified first time may include the time when a maximum blood sugar value is obtained. For example, the specified first time may include 1 hour. The specified first time may be set by the user or automatically set by the processor 220.

For example, the processor 220 may determine a time point where a specified second time elapses from a time point where the meal event is identified as a time point to measure blood sugar data of the user. The specified second time may include the time when an intermediate blood sugar value is obtained. For example, the specified second time may include 2 hours. The specified second time may be set by the user or automatically set by the processor 220.

For example, the processor 220 may determine a time point where a specified third time elapses from a time point where the meal event is identified as a time point to measure blood sugar data of the user. The specified third time may include the time when a minimum blood sugar value (e.g., fasting blood sugar value) is obtained. For example, the specified third time may include 8 hours. The specified third time may be set by the user or automatically set by the processor 220.

According to an embodiment, when the sensing value is included in a specified range corresponding to the exercise event, the processor 220 may identify that the exercise event occurs. For example, the processor 220 may determine the time point where the exercise event is identified as a time point to measure blood sugar data of the user.

According to an embodiment, the processor 220 may also determine a time point to measure blood sugar data of the user based on blood sugar values obtained from the external device 202 implemented as a continuous glucose monitoring system (CGMS) device. According to an embodiment, the processor 220 may obtain blood sugar values measured for a specified time by the external device 202 through the communication circuitry 290. According to an embodiment, when a change of the blood sugar values during the specified time is identified to be greater than a specified value, the processor 220 may identify that the meal event occurs. For example, when a change of the blood sugar values during the specified time is identified to be greater than a specified value, the processor 220 may determine that a blood sugar value of the user is increasing due to occurrence of the meal event. For example, the change of the blood sugar values during the specified time may include a value of about 1.5 mg/dl per minute. For example, the specified value may be set by the user or automatically set by the processor 220.

According to an embodiment, the processor 220 may display, through the display 260, guide information for measuring a blood sugar value of the user at a time point determined as a time point to measure blood sugar data of the user using the external device 202. According to an embodiment, the processor 220 may also output the guide information as voice.

According to an embodiment, the processor 220 may measure a sensing value indicating a biometric signal of the user at a time point determined as a time point to measure blood sugar data of the user through the first sensor 211.

According to an embodiment, the external device 202 may measure a blood sugar value of the user at a time point determined as a time point to measure blood sugar data of the user. According to an embodiment, the processor 220 may obtain, through the communication circuitry 290, information indicating a blood sugar value of the user measured by the external device 202 at a time point determined as a time point to measure blood sugar data of the user. According to an embodiment, the processor 220 may also obtain information indicating a blood sugar value of the user based on a user input.

According to an embodiment, the processor 220 may obtain calibration data based on a sensing value indicating a biometric signal measured by the first sensor 211 at a time point determined as a time point to measure blood sugar data of the user and a blood sugar value measured by the external device 202. According to an embodiment, the processor 220 may obtain a plurality of blood sugar values and a plurality of sensing values indicating biometric signals to increase reliability of the calibration data.

According to an embodiment, the processor 220 may identify a sensing value indicating a first biometric signal measured at a first time point through the first sensor 211 and identify a first blood sugar value measured at the first time point by the external device 202. The processor 220 may identify a sensing value indicating a second biometric signal measured at a second time point different from the first time point through the first sensor 211 and identify a second blood sugar value measured at the second time point by the external device 202.

According to an embodiment, the processor 220 may determine a third time point to measure a blood sugar value of the user different from the first time point and the second time point based on the first blood sugar value and the second blood sugar value. For example, the processor 220 may compare the first blood sugar value and the second blood sugar value. When the difference between the first blood sugar value and the second blood sugar value is identified to be greater than a specified value, the processor 220 may determine the third time point to obtain additional blood sugar values for obtaining the calibration data. For example, the specified value may include a difference between two adjacent blood sugar values measured by the external device 202 for obtaining the calibration curve. For example, the specified value may indicate about 50 mg/dl. For example, the specified value may be set by the user or automatically set by the processor 220.

According to an embodiment, the processor 220 may determine the third time point to measure a blood sugar value indicating a value between the first blood sugar value and the second blood sugar value. According to an embodiment, the processor 220 may determine the third time point such that a difference between a blood sugar value measured at the third time point using the external device 202 and the first blood sugar value is less than or equal to the specified value, and a difference between a blood sugar value measured at the third time point using the external device 202 and the second blood sugar value is less than or equal to the specified value.

According to an embodiment, the processor 220 may display guide information for measuring a blood sugar of the user using the external device 202 at the third time point.

According to an embodiment, the processor 220 may measure a sensing value indicating a third biometric signal through the first sensor 211 at the third time point, and identify a third blood sugar value measured by the external device 202 at the third time point.

For example, the processor 220 may determine the third time point based on reference information stored in the memory 210. The reference information may include information on a blood sugar value of a diabetes patient or information on a blood sugar value of a normal person. For example, the processor 220 may also determine the third time point based on blood sugar values obtained in real-time from the external device 202 implemented as a continuous glucose monitoring system (CGMS) device. However, this is merely an example, and the embodiments of the disclosure may determine the third time point in various ways.

According to an embodiment, the processor 220 may obtain, as the calibration data, first blood sugar data indicating a sensing value indicating a first biometric signal measured at the first time point and the first blood sugar value, second blood sugar data indicating a sensing value indicating a second biometric signal measured at the second time point and the second blood sugar value, and third blood sugar data indicating a sensing value indicating a third biometric signal measured at the third time point and the third blood sugar value. However, this is merely an example, and the number of blood sugar values may not be limited thereto.

According to an embodiment, when the calibration data is obtained, the processor 220 may terminate the function of obtaining the calibration data.

According to an embodiment, when the calibration data is obtained, the processor 220 may display, through the display 260, guide information indicating that the calibration data has been obtained. According to an embodiment, the processor 220 may also output guide information indicating that the calibration data has been obtained as voice.

According to an embodiment, after obtaining the calibration data, the processor 220 may measure a first value through the first sensor 211. For example, the first value may indicate a sensing value indicating a biometric signal of the user obtained through the first sensor 211 before determining a blood sugar value of the user using the calibration data. According to an embodiment, the processor 220 may identify a blood sugar value corresponding to the first value using the calibration data. According to an embodiment, the processor 220 may determine the identified blood sugar value as a blood sugar value of the user.

Thereafter, according to an embodiment, the processor 220 may measure a second value through the first sensor 211. For example, the second value may indicate a blood sugar value of the user obtained through the first sensor 211 before determining a blood sugar value of the user using the calibration data.

According to an embodiment, when the difference between the second value and the first value measured before the second value is identified to be greater than a specified value, the processor 220 may execute a function of updating the calibration data. According to an embodiment, executing the function of updating the calibration data may include updating (renewing) a function formula between at least one blood sugar value of the user measured by the external device 202 and at least one sensing value indicating at least one biometric signal of the user measured through the first sensor 211. For example, the specified value may indicate a value for automatically executing the function of updating the calibration data when determining that a blood sugar of the user has changed rapidly. For example, the specified value may indicate a value for automatically executing the function of updating the calibration data when determining that performance of the first sensor 211 has changed. For example, the specified value may indicate about 20 mg/dl. For example, the specified value may be set by the user or automatically set by the processor 220. According to an embodiment, when the function of updating the calibration data is executed, the processor 220 may re-perform the operation of obtaining the calibration data.

The operations of the wearable electronic device 201 described in the drawings may be performed by the processor 220. However, for convenience of description, the operations performed by the processor 220 are described as being performed by the wearable electronic device 201.

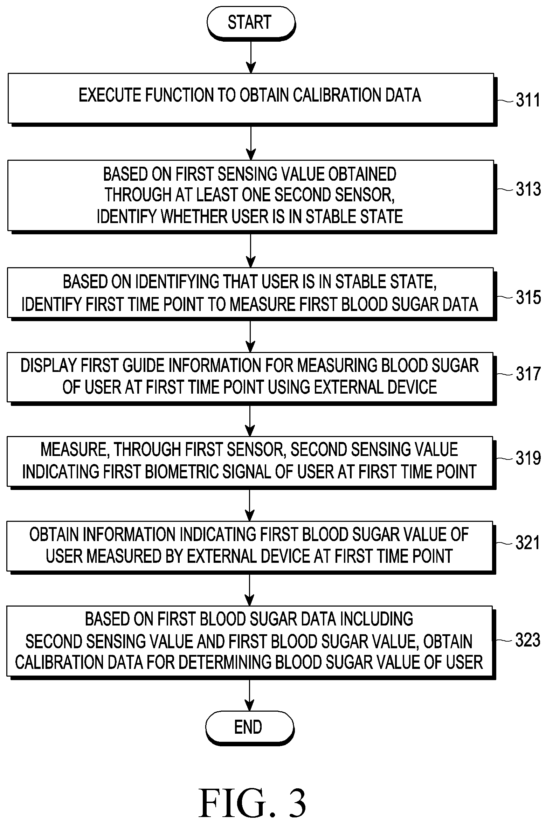

FIG. 3 is a flowchart illustrating an operation of a wearable electronic device obtaining calibration data according to an embodiment.

Referring to FIG. 3, according to an embodiment, in an operation 311, the wearable electronic device 201 (e.g., the wearable electronic device 201 of FIG. 2) may execute a function of obtaining calibration data for estimating (determining) a blood sugar value based on a sensing value indicating a biometric signal of a user to be measured using the first sensor 211 (e.g., the first sensor 211 of FIG. 2). For example, the wearable electronic device 201 may execute an application of the wearable electronic device 201 capable of executing a function of obtaining the calibration data. According to an embodiment, the calibration data may include a calibration curve indicating a relationship between at least one blood sugar value of the user measured by the external device 202 and at least one sensing value indicating at least one biometric signal of the user measured through the first sensor 211.

According to an embodiment, in an operation 313, the wearable electronic device 201 may identify whether the user is in a stable state based on a first sensing value obtained through the at least one second sensor 212 (e.g., the at least one second sensor 212 of FIG. 2). For example, the first sensing value may include at least one of a movement, a heart rate, an oxygen saturation, a stress, or a blood pressure of the user. According to an embodiment, the wearable electronic device 201 may identify whether the user is in a stable state by inputting the first sensing value into an artificial intelligence model stored in the memory 210.

According to an embodiment, in an operation 315, the wearable electronic device 201 may identify a first time point to measure first blood sugar data of a user wearing the wearable electronic device 201 based on identifying that the user is in the stable state. For example, the first blood sugar data may include a sensing value indicating a biometric signal of the user measured by the first sensor 211 at the first time point and a blood sugar value measured by the external device 202 at the first time point.

According to an embodiment, the wearable electronic device 201 may determine whether at least one of a wake-up event indicating that the user has woken up, a meal event indicating that the user is eating, or an exercise event indicating that the user is exercising is identified based on the first sensing value. For example, when the first sensing value is included in a specified range corresponding to the wake-up event, the wearable electronic device 201 may identify that the wake-up event occurs. For example, when the first sensing value is included in a specified range corresponding to the meal event, the wearable electronic device 201 may identify that the meal event occurs. For example, when the first sensing value is included in a specified range corresponding to the exercise event, the wearable electronic device 201 may identify that the exercise event occurs.

According to an embodiment, the wearable electronic device 201 may determine the first time point based on at least one of a wake-up event indicating that the user has woken up, a meal event indicating that the user is eating, or an exercise event indicating that the user is exercising. For example, the first time point may include a time point to measure a maximum blood sugar value, a minimum blood sugar value, or an intermediate blood sugar value of the user.

According to an embodiment, in an operation 317, the wearable electronic device 201 may display, through the display 260 (e.g., the display 260 of FIG. 2), first guide information for measuring a blood sugar of the user at the first time point using the external device 202. According to an embodiment, the wearable electronic device 201 may also output the first guide information as voice.

According to an embodiment, in an operation 319, the wearable electronic device 201 may measure, through the first sensor 211, a second sensing value indicating a first biometric signal of the user at the first time point. For example, the second sensing value may include a value based on an amount of light reflected from a skin of the user when light is output to the skin of the user by the first sensor 211.

According to an embodiment, in an operation 321, the wearable electronic device 201 may obtain, through the communication circuitry 290 (e.g., the communication circuitry 290 of FIG. 2), information indicating a first blood sugar value of the user measured by the external device 202 at the first time point from the external device 202. According to an embodiment, the wearable electronic device 201 may also obtain information indicating the first blood sugar value based on a user input.

According to an embodiment, in an operation 323, the wearable electronic device 201 may obtain calibration data for determining a blood sugar value of the user based on first blood sugar data including the second sensing value and the first blood sugar value. According to an embodiment, the calibration data may include a calibration curve indicating a relationship between at least one blood sugar value of the user measured by the external device 202 and at least one sensing value indicating at least one biometric signal of the user measured through the first sensor 211. For example, the calibration curve may include a function formula between at least one blood sugar value of the user measured by the external device 202 and at least one sensing value indicating at least one biometric signal of the user measured through the first sensor 211.

According to an embodiment, when a sensing value indicating a biometric signal value of the user is obtained using the first sensor 211, the wearable electronic device 201 may obtain a blood sugar value of the user corresponding to the sensing value indicating the biometric signal value using the calibration curve. The wearable electronic device 201 may estimate (determine) the blood sugar value of the user obtained using the calibration curve as the blood sugar value of the user.

According to an embodiment, a blood sugar value based on a sensing value indicating a biometric signal value measured by the first sensor 211 of the wearable electronic device 201 may be less accurate than a blood sugar value measured in an invasive manner by the external device 202 because an amount of light reflected from the skin may vary according to a skin color of the user. A method of measuring a blood sugar of the user in an invasive manner using the external device 202 may be more accurate than a method of measuring a blood sugar of the user in a non-invasive manner, but may cause pain to the user. According to an embodiment, the wearable electronic device 201 may compensate a blood sugar value based on a biometric signal of the user measured in a non-invasive manner through the first sensor 211 using the calibration data. According to an embodiment, the wearable electronic device 201 may determine the compensated blood sugar value as the blood sugar value of the user. Accordingly, the wearable electronic device 201 may provide an accurate blood sugar value to the user.

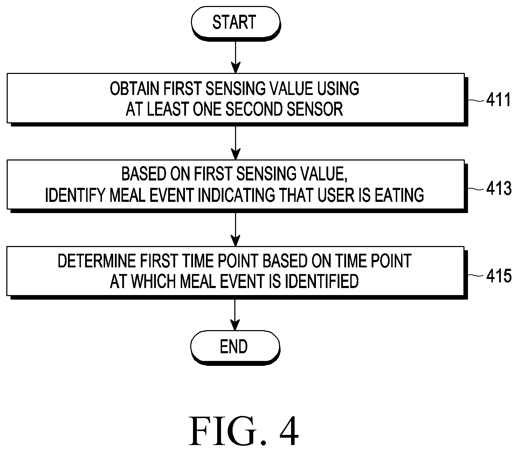

FIG. 4 is a flowchart illustrating an operation of a wearable electronic device determining a time point to measure blood sugar data based on a meal event according to an embodiment.

Referring to FIG. 4, according to an embodiment, in an operation 411, the wearable electronic device 201 (e.g., the wearable electronic device 201 of FIG. 2) may obtain a first sensing value using the at least one second sensor 212 (e.g., the at least one second sensor 212 of FIG. 2). For example, the first sensing value may include a sensing value indicating at least one of a movement, a heart rate, an oxygen saturation, a stress, or a blood pressure of the user.

According to an embodiment, in an operation 413, the wearable electronic device 201 may identify a meal event indicating that the user is eating based on the first sensing value. For example, the wearable electronic device 201 may identify the meal event by analyzing a sensing value indicating a movement of the user using an artificial intelligence model stored in the memory 210. The artificial intelligence model may include a temporal convolution net (TCN), a decision tree (DT), or a k-nearest neighbor (KNN).

According to an embodiment, the wearable electronic device 201 may also identify the meal event based on a voice signal obtained through a microphone included in the wearable electronic device 201. In this case, the wearable electronic device 201 may also identify the meal event by further considering a voice signal obtained from an external electronic device wearable on an ear of the user.

According to an embodiment, in an operation 415, the wearable electronic device 201 may determine a first time point to measure blood sugar data of the user based on a time point where the meal event is identified.

According to an embodiment, the wearable electronic device 201 may determine a time point where a specified first time elapses from a time point where the meal event is identified as the first time point. The specified first time may include the time when a maximum blood sugar value is obtained. For example, the specified first time may include 1 hour.

According to an embodiment, the wearable electronic device 201 may determine a time point where a specified second time elapses from a time point where the meal event is identified as the first time point. The specified second time may include the time when an intermediate blood sugar value is obtained. For example, the specified second time may include 2 hours.

According to an embodiment, the wearable electronic device 201 may determine a time point where a specified third time elapses from a time point where the meal event is identified as the first time point. The specified third time may include the time when a minimum blood sugar value (e.g., fasting blood sugar value) is obtained. For example, the specified third time may include 8 hours.

According to an embodiment, the wearable electronic device 201 may display, through the display 260 (e.g., the display 260 of FIG. 2), guide information for measuring a blood sugar value of the user at the first time point using the external device 202 (e.g., the external device 202 of FIG. 2). According to an embodiment, the wearable electronic device 201 may identify a second sensing value indicating a first biometric signal of the user measured at the first time point through the first sensor 211 (e.g., the first sensor 211 of FIG. 2), and identify a first blood sugar value of the user measured by the external device 202 at the first time point through the communication circuitry 290 (e.g., the communication circuitry 290 of FIG. 2). According to an embodiment, the wearable electronic device 201 may obtain first blood sugar data including the second sensing value indicating the first biometric signal and the first blood sugar value as calibration data for estimating (determining) a blood sugar value of the user.

Accordingly, the wearable electronic device 201 may obtain a maximum blood sugar value, an intermediate blood sugar value, or a minimum blood sugar value of the user measured by the first sensor 211 and the external device 202 at the first time point as the calibration data.

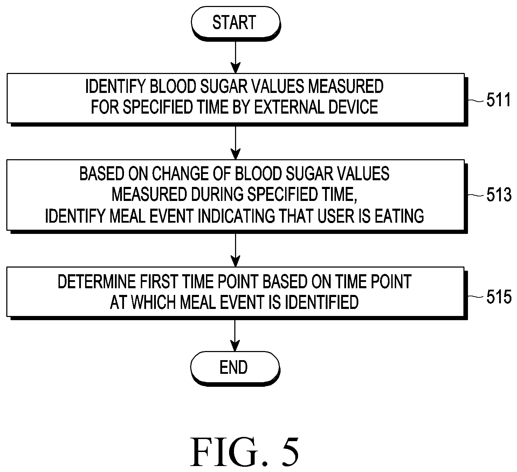

FIG. 5 is a flowchart illustrating an operation of a wearable electronic device determining a time point to measure blood sugar data based on a meal event according to an embodiment.

Referring to FIG. 5, according to an embodiment, in an operation 511, the wearable electronic device 201 (e.g., the wearable electronic device 201 of FIG. 2) may identify blood sugar values measured for a specified time by the external device 202 (e.g., the external device 202 of FIG. 2) through the communication circuitry 290 (e.g., the communication circuitry 290 of FIG. 2). For example, the external device 202 may be implemented as a continuous glucose monitoring system (CGMS) device. For example, the wearable electronic device 201 may obtain blood sugar values measured by the external device 202 in real-time.

According to an embodiment, in an operation 513, the wearable electronic device 201 may identify a meal event indicating that the user is eating based on a change of the blood sugar values measured for the specified time. According to an embodiment, the wearable electronic device 201 may compare the change of the blood sugar values during the specified time and a specified value. According to an embodiment, the wearable electronic device 201 may identify the meal event based on identifying that the change of the blood sugar values during the specified time is greater than a specified value. For example, when the change of the blood sugar values during the specified time is identified to be greater than the specified value, the wearable electronic device 201 may determine that a blood sugar value of the user is increasing due to occurrence of the meal event. For example, the change of the blood sugar values during the specified time may include a value of about 1.5 mg/dl per minute.

According to an embodiment, in an operation 515, the wearable electronic device 201 may determine the first time point based on a time point where the meal event is identified.

According to an embodiment, the wearable electronic device 201 may determine a time point where a specified first time elapses from a time point where the meal event is identified as the first time point. The specified first time may include the time when a maximum blood sugar value is obtained. For example, the specified first time may include 1 hour. According to an embodiment, the wearable electronic device 201 may also determine a time point where a specified second time elapses from a time point where the meal event is identified as the first time point. The specified second time may include the time when an intermediate blood sugar value is obtained. For example, the specified second time may include 2 hours. According to an embodiment, the wearable electronic device 201 may also determine a time point where a specified third time elapses from a time point where the meal event is identified as the first time point. The specified third time may include the time when a minimum blood sugar value (e.g., fasting blood sugar value) is obtained. For example, the specified third time may include 8 hours.

According to an embodiment, the wearable electronic device 201 may display, through the display 260 (e.g., the display 260 of FIG. 2), guide information for measuring a blood sugar value of the user at the first time point using the external device 202 (e.g., the external device 202 of FIG. 2). According to an embodiment, the wearable electronic device 201 may identify a second sensing value indicating a first biometric signal of the user measured at the first time point through the first sensor 211 (e.g., the first sensor 211 of FIG. 2), and identify a first blood sugar value of the user measured by the external device 202 at the first time point through the communication circuitry 290 (e.g., the communication circuitry 290 of FIG. 2). According to an embodiment, the wearable electronic device 201 may obtain first blood sugar data including the first blood sugar value and the second sensing value as the calibration data.

Accordingly, the wearable electronic device 201 may obtain a maximum blood sugar value, an intermediate blood sugar value, or a minimum blood sugar value of the user measured by the first sensor 211 and the external device 202 at the first time point as the calibration data.

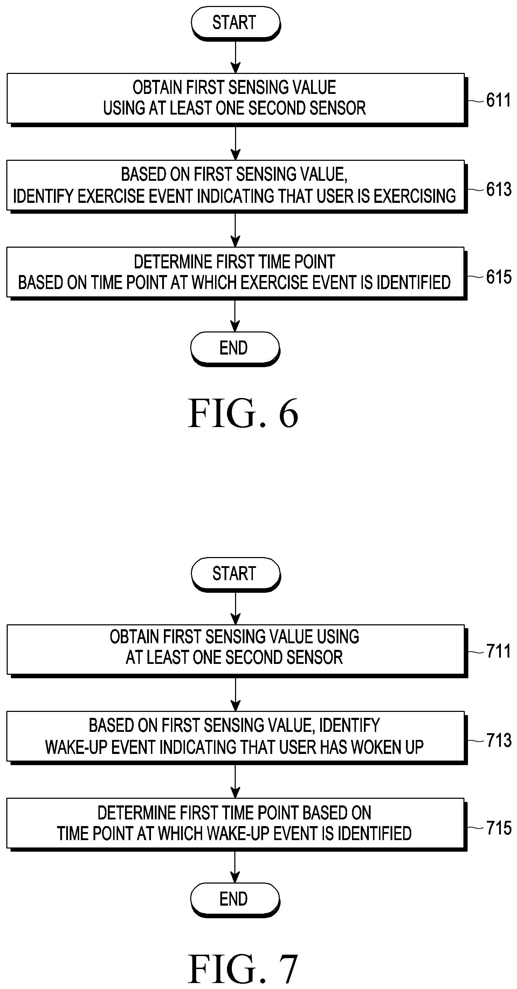

FIG. 6 is a flowchart illustrating an operation of a wearable electronic device determining a time point to measure blood sugar data based on an exercise event according to an embodiment.

Referring to FIG. 6, according to an embodiment, in an operation 611, the wearable electronic device 201 (e.g., the wearable electronic device 201 of FIG. 2) may obtain a first sensing value using the at least one second sensor 212 (e.g., the at least one second sensor 212 of FIG. 2). For example, the first sensing value may include a sensing value indicating at least one of a movement, a heart rate, an oxygen saturation, a stress, or a blood pressure of the user.

According to an embodiment, in an operation 613, the wearable electronic device 201 may identify an exercise event indicating that the user is exercising based on the first sensing value. For example, when the first sensing value is included in a specified range corresponding to the exercise event, the wearable electronic device 201 may identify that the exercise event occurs.

According to an embodiment, in an operation 615, the wearable electronic device 201 may determine the first time point based on a time point where the exercise event is identified. For example, the wearable electronic device 201 may determine a time point where the exercise event is identified as a time point to measure blood sugar data of the user.

According to an embodiment, the wearable electronic device 201 may display, through the display 260 (e.g., the display 260 of FIG. 2), guide information for measuring a blood sugar value of the user at the first time point using the external device 202 (e.g., the external device 202 of FIG. 2). According to an embodiment, the wearable electronic device 201 may identify a second sensing value indicating a first biometric signal of the user measured at the first time point through the first sensor 211 (e.g., the first sensor 211 of FIG. 2), and identify a first blood sugar value of the user measured by the external device 202 at the first time point through the communication circuitry 290 (e.g., the communication circuitry 290 of FIG. 2). According to an embodiment, the wearable electronic device 201 may obtain first blood sugar data including the second sensing value and the first blood sugar value as the calibration data.

Accordingly, the wearable electronic device 201 may obtain an intermediate blood sugar value of the user measured by the first sensor 211 and the external device 202 at the first time point where the exercise event is identified as the calibration data.

FIG. 7 is a flowchart illustrating an operation of a wearable electronic device determining a time point to measure blood sugar data based on a wake-up event according to an embodiment.

Referring to FIG. 7, according to an embodiment, in an operation 711, the wearable electronic device 201 (e.g., the wearable electronic device 201 of FIG. 2) may obtain a first sensing value using the at least one second sensor 212 (e.g., the at least one second sensor 212 of FIG. 2). For example, the first sensing value may include a sensing value indicating at least one of a movement, a heart rate, an oxygen saturation, a stress, or a blood pressure of the user.

According to an embodiment, in an operation 713, the wearable electronic device 201 may identify a wake-up event indicating that the user has woken up based on the first sensing value. For example, when the sensing value is included in a specified range corresponding to the wake-up event, the wearable electronic device 201 may identify that the wake-up event occurs.

According to an embodiment, in an operation 715, the wearable electronic device 201 may determine the first time point based on a time point where the wake-up event is identified. For example, the wearable electronic device 201 may determine a time point where the wake-up event is identified as a time point to measure blood sugar data of the user.

According to an embodiment, the wearable electronic device 201 may display, through the display 260 (e.g., the display 260 of FIG. 2), guide information for measuring a blood sugar value of the user at the first time point using the external device 202 (e.g., the external device 202 of FIG. 2). According to an embodiment, the wearable electronic device 201 may identify a second sensing value indicating a first biometric signal of the user measured at the first time point through the first sensor 211 (e.g., the first sensor 211 of FIG. 2), and identify a first blood sugar value of the user measured by the external device 202 at the first time point through the communication circuitry 290 (e.g., the communication circuitry 290 of FIG. 2). According to an embodiment, the wearable electronic device 201 may obtain first blood sugar data including the second sensing value indicating the first biometric signal and the first blood sugar value as the calibration data.

Accordingly, the wearable electronic device 201 may obtain a minimum blood sugar value of the user measured by the first sensor 211 and the external device 202 at a time point where the wake-up event is identified as the calibration data.

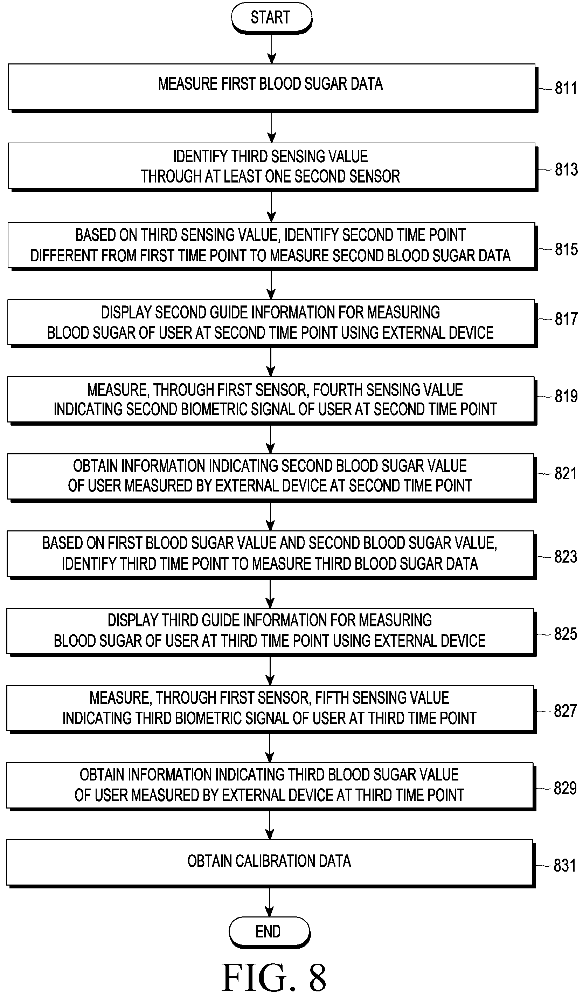

FIG. 8 is a flowchart illustrating an operation of a wearable electronic device obtaining calibration data according to an embodiment.

Referring to FIG. 8, according to an embodiment, in an operation 811, the wearable electronic device 201 (e.g., the wearable electronic device 201 of FIG. 2) may measure first blood sugar data. For example, the wearable electronic device 201 may measure a second sensing value indicating a first biometric signal at the first time point through the first sensor 211 (e.g., the first sensor 211 of FIG. 2), and identify a first blood sugar value measured at the first time point from the external device 202 (e.g., the external device 202 of FIG. 2).

According to an embodiment, in an operation 813, the wearable electronic device 201 may identify a third sensing value through the at least one second sensor 212 (e.g., the at least one second sensor 212 of FIG. 2). For example, the third sensing value may include a sensing value indicating at least one of a movement, a heart rate, an oxygen saturation, a stress, or a blood pressure of the user.

According to an embodiment, in an operation 815, the wearable electronic device 201 may identify a second time point different from the first time point to measure second blood sugar data based on the third sensing value. According to an embodiment, the wearable electronic device 202 may identify whether the user is in a stable state based on the third sensing value. According to an embodiment, the wearable electronic device 202 may identify the second time point based on identifying that the user is in the stable state. For example, the second time point may be determined based on a meal event, an exercise event, or a wake-up event. For example, the second blood sugar data may include a sensing value indicating a biometric signal measured by the first sensor 211 (e.g., the first sensor 211 of FIG. 2) at the second time point and a blood sugar value measured by the external device 202 at the second time point.

According to an embodiment, in an operation 817, the wearable electronic device 201 may display, through the display 260 (e.g., the display 260 of FIG. 2), second guide information for measuring a blood sugar of the user using the external device 202 at the second time point.

According to an embodiment, in an operation 819, the wearable electronic device 201 may measure, through the first sensor 211, a fourth sensing value indicating a second biometric signal of the user at the second time point.

According to an embodiment, in an operation 821, the wearable electronic device 201 may obtain, through the communication circuitry 290 (e.g., the communication circuitry 290 of FIG. 2), information indicating a second blood sugar value of the user measured by the external device 202 at the second time point.

According to an embodiment, in an operation 823, the wearable electronic device 201 may identify a third time point to measure third blood sugar data based on the first blood sugar value and the second blood sugar value. For example, the third blood sugar data may include a sensing value indicating a third biometric signal measured by the first sensor 211 at the third time point and a blood sugar value measured by the external device 202 at the third time point.

According to an embodiment, the wearable electronic device 201 may compare the first blood sugar value and the second blood sugar value. According to an embodiment, when the difference between the first blood sugar value and the second blood sugar value is identified to be greater than a specified value, the wearable electronic device 201 may determine the third time point to obtain additional third blood sugar data for obtaining the calibration data. For example, the specified value may include a difference between two adjacent blood sugar values measured by the external device 202 for obtaining the calibration curve. For example, the specified value may include about 50 mg/dl. For example, the specified value may be set by the user or automatically set by the processor 220.

According to an embodiment, the wearable electronic device 201 may determine the third time point to measure a blood sugar value indicating a value between the first blood sugar value and the second blood sugar value. According to an embodiment, the wearable electronic device 201 may determine the third time point such that a difference between a blood sugar value measured at the third time point using the external device 202 and the second blood sugar value is less than or equal to a specified value, and a difference between a blood sugar value measured at the third time point using the external device 202 and the second blood sugar value is less than or equal to a specified value.

For example, the wearable electronic device 201 may determine the third time point based on reference information stored in the memory 210 (e.g., the memory 210 of FIG. 2). The reference information may include a blood sugar value of a diabetes patient or a blood sugar value of a normal person. For example, the wearable electronic device 201 may also determine the third time point based on blood sugar values obtained in real-time from the external device 202 implemented as a continuous glucose monitoring system (CGMS) device. However, this is merely an example, and the embodiments of the disclosure may determine the third time point in various ways.

According to an embodiment, in an operation 825, the wearable electronic device 201 may display, through the display 260, third guide information for measuring a blood sugar of the user using the external device 202 at the third time point.

According to an embodiment, in an operation 827, the wearable electronic device 201 may measure, through the first sensor 211, a fifth sensing value indicating a third biometric signal of the user at the third time point.

According to an embodiment, in an operation 829, the wearable electronic device 201 may obtain information indicating a third blood sugar value of the user measured by the external device 202 at the third time point.

According to an embodiment, in an operation 831, the wearable electronic device 201 may obtain calibration data based on the first blood sugar data, the second blood sugar data, and the third blood sugar data. According to an embodiment, the calibration data may include a calibration curve indicating a relationship between at least one blood sugar value of the user measured by the external device 202 and at least one sensing value indicating at least one biometric signal of the user measured through the first sensor 211. For example, the calibration curve may include a function formula between at least one blood sugar value of the user measured by the external device 202 and at least one sensing value indicating at least one biometric signal of the user measured through the first sensor 211.

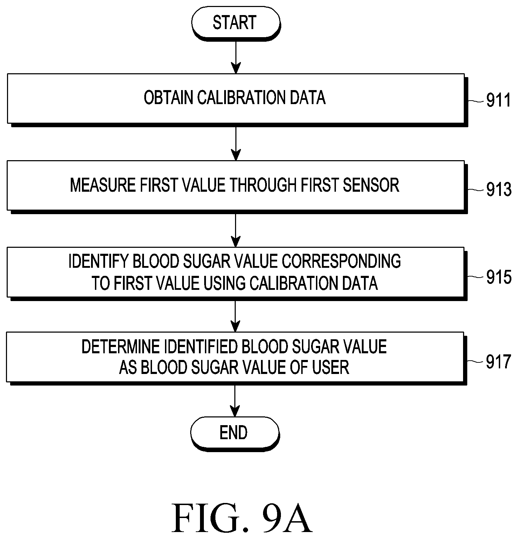

FIG. 9A is a flowchart illustrating an operation of a wearable electronic device determining a blood sugar value based on a sensing value measured through a first sensor using calibration data according to an embodiment.

Referring to FIG. 9A, according to an embodiment, in an operation 911, the wearable electronic device 201 (e.g., the wearable electronic device 201 of FIG. 2) may obtain calibration data. According to an embodiment, the calibration data may include a calibration curve indicating a relationship between at least one blood sugar value of the user measured by the external device 202 (e.g., the external device 202 of FIG. 2) and at least one sensing value indicating at least one biometric signal of the user measured through the first sensor 211 (e.g., the first sensor 211 of FIG. 2). For example, the calibration curve may include a function formula between at least one blood sugar value of the user measured by the external device 202 and at least one sensing value indicating at least one biometric signal of the user measured through the first sensor 211. According to an embodiment, after obtaining the calibration data, the wearable electronic device 201 may terminate a function of obtaining the calibration data.

According to an embodiment, in an operation 913, the wearable electronic device 201 may measure, through the first sensor 211, a first value indicating a biometric signal of the user. For example, the first value may include a sensing value based on an amount of light reflected from a skin of the user when light is output to the skin of the user by the first sensor 211.

According to an embodiment, in an operation 915, the wearable electronic device 201 may identify a blood sugar value corresponding to the first value using the calibration data.

According to an embodiment, in an operation 917, the wearable electronic device 201 may determine the identified blood sugar value as a blood sugar value of the user.

According to an embodiment, a blood sugar value based on a sensing value indicating a biometric signal measured by the first sensor 211 of the wearable electronic device 201 may be less accurate than a blood sugar value measured in an invasive manner by the external device 202 because an amount of light reflected from the skin may vary according to a skin color of the user. A method of measuring a blood sugar of the user in an invasive manner using the external device 202 may be more accurate than a method of measuring a blood sugar of the user in a non-invasive manner, but may cause pain to the user. According to an embodiment, the wearable electronic device 201 may identify a blood sugar value corresponding to a biometric signal value of the user measured in a non-invasive manner through the first sensor 211 using the calibration data and determine the blood sugar value as a blood sugar value of the user. Accordingly, the wearable electronic device 201 may provide an accurate blood sugar value to the user.

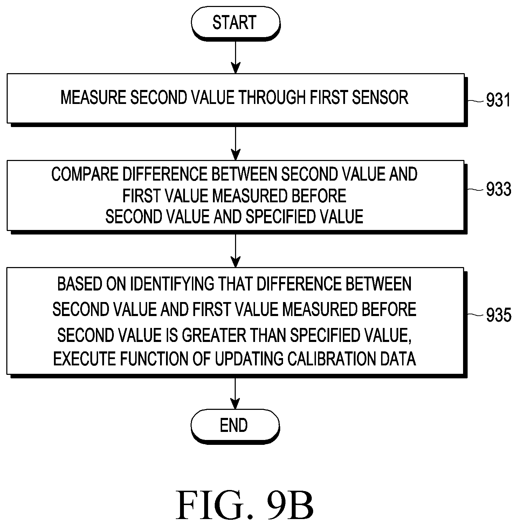

FIG. 9B is a flowchart illustrating an operation of a wearable electronic device updating calibration data according to an embodiment.

Referring to FIG. 9B, according to an embodiment, in an operation 931, the wearable electronic device 201 (e.g., the wearable electronic device 201 of FIG. 2) may measure, through the first sensor 211 (e.g., the first sensor 211 of FIG. 2), a second value indicating a biometric signal of the user. For example, the second value may include a sensing value based on an amount of light reflected from a skin of the user when light is output to the skin of the user by the first sensor 211.

According to an embodiment, in an operation 933, the wearable electronic device 201 may compare a difference between the second value and a first value indicating a biometric signal value of the user measured through the first sensor 211 before the second value and a specified value. For example, the specified value may indicate a value for executing a function of updating the calibration data when determining that a sensing value indicating a biometric signal of the user has changed rapidly. For example, the specified value may indicate a value for executing a function of updating the calibration data when determining that performance of the first sensor 211 has changed. For example, the specified value may indicate about 20 mg/dl.

According to an embodiment, in an operation 935, the wearable electronic device 201 may execute a function of updating the calibration data based on identifying that a difference between the second value and the first value measured before the second value is greater than a specified value. According to an embodiment, when the function of updating the calibration data is executed, the wearable electronic device 201 may re-perform the operation of obtaining the calibration data.

According to an embodiment, the calibration data may include a calibration curve indicating a relationship between at least one blood sugar value of the user measured by the external device 202 and at least one sensing value indicating at least one biometric signal of the user measured through the first sensor 211. For example, the calibration curve may include a function formula between at least one blood sugar value of the user measured by the external device 202 and at least one sensing value indicating at least one biometric signal of the user measured through the first sensor 211. According to an embodiment, executing the function of updating the calibration data may include updating a function formula between at least one blood sugar value of the user measured by the external device 202 and at least one sensing value indicating at least one biometric signal of the user measured through the first sensor 211.

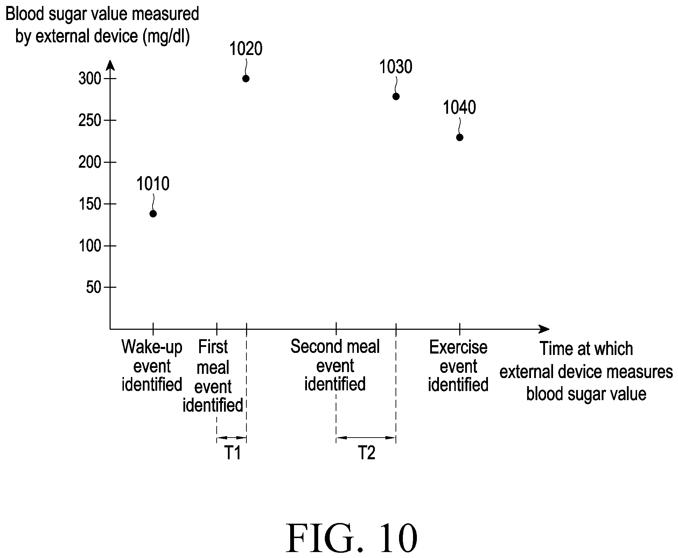

FIG. 10 is a view illustrating information on a blood sugar value measured by an external device according to an embodiment.

Referring to FIG. 10, according to an embodiment, a horizontal axis of the graph may indicate the time when the external device 202 (e.g., the external device 202 of FIG. 2) measures a blood sugar value, and a vertical axis may indicate a blood sugar value measured by the external device 202.

According to an embodiment, the wearable electronic device 201 (e.g., the wearable electronic device 201 of FIG. 2) may determine a time point to measure blood sugar data of the user based on a sensing value obtained through the at least one second sensor 212 (e.g., the at least one second sensor 212 of FIG. 2). For example, the sensing value may include at least one of a movement, a heart rate, an oxygen saturation, a stress, or a blood pressure of the user. For example, the time point may include a time point to measure a maximum blood sugar value, a minimum blood sugar value, or an intermediate blood sugar value of the user. For example, the time point may be determined based on at least one of a wake-up event indicating that the user has woken up, a meal event indicating that the user is eating, or an exercise event indicating that the user is exercising.

According to an embodiment, the wearable electronic device 201 may identify a wake-up event indicating that the user has woken up. According to an embodiment, the wearable electronic device 201 may determine a time point where the wake-up event is identified as a time point to measure blood sugar data. According to an embodiment, the wearable electronic device 201 may obtain, through the communication circuitry 290 (e.g., the communication circuitry 290 of FIG. 2), a blood sugar value 1010 measured by the external device 202 at a time point where the wake-up event is identified. For example, the blood sugar value 1010 may indicate about 140 mg/dl.

According to an embodiment, the wearable electronic device 201 may identify a first meal event indicating that the user is eating. According to an embodiment, the wearable electronic device 201 may determine a time point where a specified first time T1 elapses from a time point where the first meal event is identified as a time point to measure blood sugar data of the user. The specified first time T1 may include the time when a maximum blood sugar value is obtained. For example, the specified first time may include 1 hour. According to an embodiment, the wearable electronic device 201 may obtain, through the communication circuitry 290, a blood sugar value 1020 measured by the external device 202 at a time point where the specified first time T1 elapses from a time point where the first meal event is identified. For example, the blood sugar value 1020 may indicate about 300 mg/dl.

According to an embodiment, the wearable electronic device 201 may identify a second meal event indicating that the user is eating. According to an embodiment, the wearable electronic device 201 may determine a time point where a specified second time T2 elapses from a time point where the second meal event is identified as a time point to measure blood sugar data of the user. The specified second time T2 may include the time when an intermediate blood sugar value is obtained. For example, the specified second time may include 2 hours. According to an embodiment, the wearable electronic device 201 may obtain, through the communication circuitry 290, a blood sugar value 1030 measured by the external device 202 at a time point where the specified second time T2 elapses from a time point where the second meal event is identified. For example, the blood sugar value 1030 may indicate about 270 mg/dl.

According to an embodiment, the wearable electronic device 201 may identify an exercise event indicating that the user is exercising. According to an embodiment, the wearable electronic device 201 may determine a time point to measure blood sugar data of the user based on the exercise event. For example, the wearable electronic device 201 may determine a time point where the exercise event is identified as a time point to measure blood sugar data of the user. The time point where the exercise event is identified may include the time when an intermediate blood sugar value is obtained. According to an embodiment, the wearable electronic device 201 may obtain, through the communication circuitry 290, a blood sugar value 1040 measured by the external device 202 at a time point where the exercise event is identified. For example, the blood sugar value 1040 may indicate about 230 mg/dl.

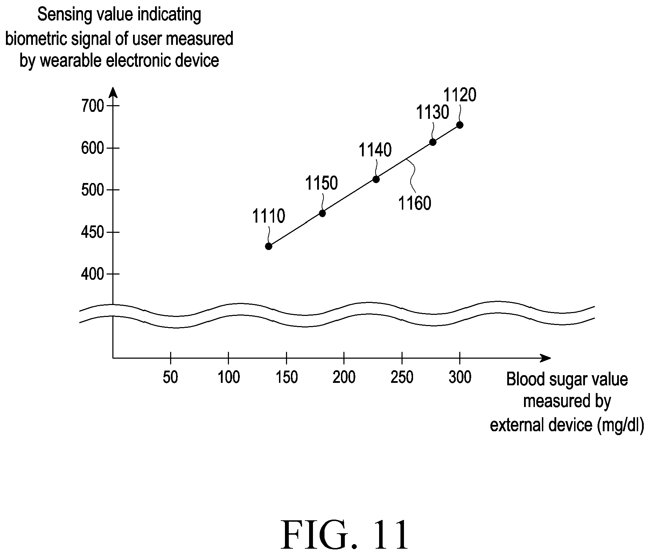

FIG. 11 is a view illustrating an operation of a wearable electronic device obtaining calibration data according to an embodiment.

Referring to FIG. 11 and FIG. 10, the calibration data may include a calibration curve 1160 indicating a relationship between blood sugar values measured by the external device 202 (e.g., the external device 202 of FIG. 2) and sensing values indicating biometric signals measured by the wearable electronic device 201 (e.g., the wearable electronic device 201 of FIG. 2). For example, the sensing values indicating the biometric signals may include values based on an amount of light reflected from a skin of the user when light is output to the skin of the user by the first sensor 211.

According to an embodiment, a horizontal axis of the calibration curve 1160 may indicate a blood sugar value measured by the external device 202, and a vertical axis may indicate a sensing value indicating biometric signals of the user measured by the wearable electronic device 201.

According to an embodiment, the wearable electronic device 201 (e.g., the wearable electronic device 201 of FIG. 2) may measure a sensing value indicating a biometric signal of the user at a time point where the wake-up event is identified through the first sensor 211 (e.g., the first sensor 211 of FIG. 2). For example, the sensing value indicating a biometric signal measured by the first sensor 211 may indicate about 425. For example, the blood sugar value 1010 (e.g., the blood sugar value 1010 of FIG. 10) measured by the external device 202 may indicate about 140 mg/dl. According to an embodiment, the wearable electronic device 201 may obtain first blood sugar data 1110 based on the blood sugar value 1010 measured by the external device 202 and the sensing value indicating the biometric signal measured by the first sensor 211.

According to an embodiment, the wearable electronic device 201 may measure a blood sugar value at a time point where the specified first time T1 elapses from a time point where the first meal event is identified through the first sensor 211. For example, the sensing value indicating a biometric signal measured by the first sensor 211 may indicate about 650. For example, the blood sugar value 1020 (e.g., the blood sugar value 1020 of FIG. 10) measured by the external device 202 may indicate about 300 mg/dl. According to an embodiment, the wearable electronic device 201 may obtain second blood sugar data 1120 based on the blood sugar value 1020 measured by the external device 202 and the sensing value measured by the first sensor 211.

According to an embodiment, the wearable electronic device 201 may measure a blood sugar value at a time point where the specified second time T2 elapses from a time point where the second meal event is identified through the first sensor 211. For example, the sensing value indicating a biometric signal measured by the first sensor 211 may indicate about 620. For example, the blood sugar value 1030 (e.g., the blood sugar value 1030 of FIG. 10) measured by the external device 202 may indicate about 270 mg/dl. According to an embodiment, the wearable electronic device 201 may obtain third blood sugar data 1130 based on the blood sugar value 1030 measured by the external device 202 and the sensing value measured by the first sensor 211.

According to an embodiment, the wearable electronic device 201 may measure a blood sugar value at a time point where the exercise event is identified through the first sensor 211. For example, the sensing value indicating a biometric signal measured by the first sensor 211 may indicate about 500. For example, the blood sugar value 1040 (e.g., the blood sugar value 1040 of FIG. 10) measured by the external device 202 may indicate about 230 mg/dl. According to an embodiment, the wearable electronic device 201 may obtain fourth blood sugar data 1140 based on the blood sugar value 1040 measured by the external device 202 and the sensing value measured by the first sensor 211.

According to an embodiment, as a result of comparing the blood sugar value 1010 measured by the external device 202 included in the first blood sugar data 1110 and the blood sugar value 1040 measured by the external device 202 included in the fourth blood sugar data 1140, the wearable electronic device 201 may identify that a difference (e.g., 70 mg/dl) between the blood sugar value 1010 and the blood sugar value 1040 is greater than a specified value. According to an embodiment, based on identifying that a difference between the blood sugar value 1010 and the blood sugar value 1040 is greater than a specified value, the wearable electronic device 201 may determine a specific time point to obtain additional blood sugar data for obtaining the calibration data. For example, the specified value may include a difference between two adjacent blood sugar values measured by the external device 202 for obtaining the calibration curve 1160. For example, the specified value may indicate 50 mg/dl. According to an embodiment, the wearable electronic device 201 may determine the specific time point such that a difference between a blood sugar value measured at the determined specific time point using the external device 202 and the blood sugar value 1010 is less than or equal to a specified value, and a difference between a blood sugar value measured at the determined specific time point using the external device 202 and the blood sugar value 1040 is less than or equal to a specified value.

According to an embodiment, the wearable electronic device 201 may display, through the display 260 (e.g., the display 260 of FIG. 2), guide information for measuring a blood sugar value of the user at the specific time point using the external device 202.

According to an embodiment, the wearable electronic device 201 may measure a sensing value indicating a biometric signal of the user at the specific time point through the first sensor 211. According to an embodiment, the wearable electronic device 201 may obtain, through the communication circuitry 290, a blood sugar value measured by the external device 202 at the specific time point. According to an embodiment, the wearable electronic device 201 may obtain fifth blood sugar data 1150 based on a blood sugar value measured by the external device 202 at the specific time point and a sensing value measured by the first sensor 211 at the specific time point. For example, the sensing value indicating a biometric signal of the user measured through the first sensor 211 may indicate about 470, and a blood sugar value of the user measured by the external device 202 may indicate about 190 mg/dl.

According to an embodiment, the wearable electronic device 201 may obtain calibration data (e.g., the calibration curve 1160) using the blood sugar data 1110, 1120, 1130, 1140, 1150.

For example, the calibration curve may include a function formula between at least one blood sugar value of the user measured by the external device 202 and at least one sensing value indicating at least one biometric signal value of the user measured through the first sensor 211. For convenience of description, although the calibration curve is illustrated as being implemented as a first-order function in FIG. 11, the calibration curve may be implemented as an nth order (n is a natural number greater than 1) function.

According to an embodiment, the embodiments of the disclosure may not be limited to the number of blood sugar values and the numerical values of the blood sugar values illustrated in FIG. 11.

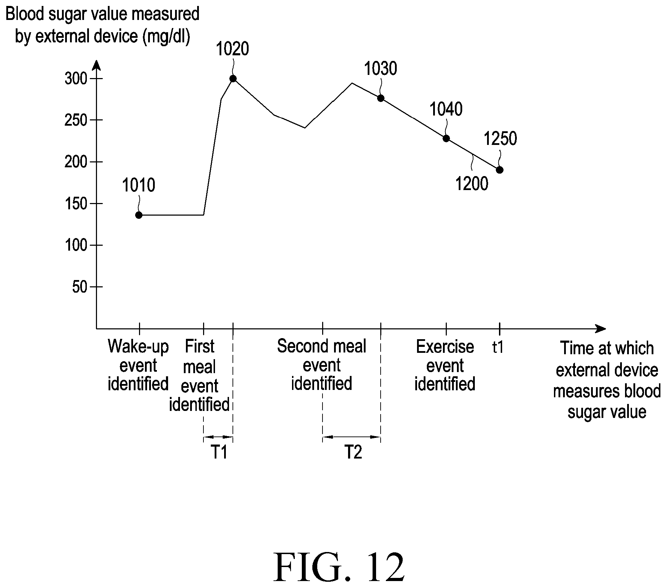

FIG. 12 is a view illustrating information on a blood sugar value measured by an external device implemented as a continuous glucose monitoring system according to an embodiment.

Referring to FIG. 12, according to an embodiment, the external device 202 (e.g., the external device 202 of FIG. 2) may be implemented as a continuous glucose monitoring system (CGMS) device. According to an embodiment, the wearable electronic device 201 (e.g., the wearable electronic device 201 of FIG. 2) may receive blood sugar values 1200 measured by the external device 202 in real-time through the communication circuitry 290 (e.g., the communication circuitry 290 of FIG. 2).

A horizontal axis of the graph illustrated in FIG. 12 may indicate the time when the external device 202 measures a blood sugar value, and a vertical axis may indicate a blood sugar value measured by the external device 202.

According to an embodiment, the wearable electronic device 201 may identify a blood sugar value 1010 received from the external device 202 at a time point where the wake-up event is identified.

According to an embodiment, the wearable electronic device 201 may identify a blood sugar value 1020 received from the external device 202 at a time point where the specified first time T1 elapses from a time point where the first meal event is identified.

According to an embodiment, the wearable electronic device 201 may identify a blood sugar value 1030 received from the external device 202 at a time point where the specified second time T2 elapses from a time point where the second meal event is identified.

According to an embodiment, the wearable electronic device 201 may identify a blood sugar value 1040 received from the external device 202 at a time point where the exercise event is identified.

According to an embodiment, the wearable electronic device 201 may identify that a difference between the blood sugar value 1010 obtained at a time point where the wake-up event is identified and the blood sugar value 1040 obtained at a time point where the exercise event is identified is greater than a specified value. In this case, the wearable electronic device 201 may determine a t1 time point to measure additional blood sugar data for obtaining the calibration data.

According to an embodiment, the wearable electronic device 201 may determine the t1 time point by analyzing blood sugar values 1200 received in real-time by the continuous glucose monitoring system (CGMS) device. The blood sugar value 1250 received from the external electronic device 202 at the t1 time point may be a value between the blood sugar value 1010 obtained at a time point where the wake-up event is identified and the blood sugar value 1040 obtained at a time point where the exercise event is identified, and a difference between the blood sugar value 1010 obtained at a time point where the wake-up event is identified may be not greater than a specified value, and a difference between the blood sugar value 1040 obtained at a time point where the exercise event is identified may be not greater than a specified value.

According to an embodiment, the wearable electronic device 201 may measure a blood sugar value of the user at the t1 time point using the first sensor 211 (e.g., the first sensor 211 of FIG. 2). According to an embodiment, the wearable electronic device 201 may identify the blood sugar value 1250 received from the external electronic device 202 at the t1 time point, and measure a blood sugar value of the user at the t1 time point using the first sensor 211.

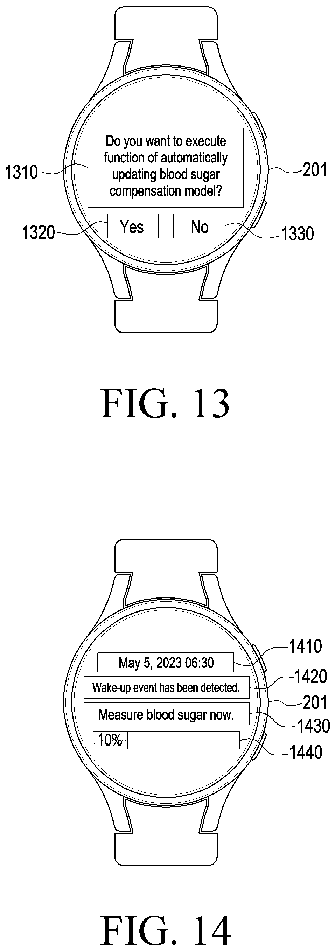

FIG. 13 is a view illustrating a screen displayed through a display when a wearable electronic device executes a function of obtaining calibration data according to an embodiment.

Referring to FIG. 13, according to an embodiment, the wearable electronic device 201 (e.g., the wearable electronic device 201 of FIG. 2) may execute, through the display 260 (e.g., the display 260 of FIG. 2), an application capable of executing a function of obtaining calibration data.

According to an embodiment, when the application is executed, the wearable electronic device 201 may display information 1310 (e.g., "Do you want to execute a function of automatically updating a blood sugar compensation model?") indicating whether to execute a function of obtaining calibration data on an execution screen of the application.

According to an embodiment, when a user input for a first object 1320 indicating to execute a function of obtaining calibration data is identified, the wearable electronic device 201 may execute a function of obtaining calibration data.

According to an embodiment, when a user input for a second object 1330 indicating not to execute a function of obtaining calibration data is identified, the wearable electronic device 201 may not execute a function of obtaining calibration data.

FIG. 14 is a view illustrating a screen displayed through a display when a wearable electronic device identifies a wake-up event according to an embodiment.

Referring to FIG. 14, according to an embodiment, the wearable electronic device 201 (e.g., the wearable electronic device 201 of FIG. 2) may obtain a sensing value through the at least one second sensor 212 (e.g., the at least one second sensor 212 of FIG. 2). For example, the sensing value may include a sensing value indicating at least one of a heart rate, an oxygen saturation, a stress, or a blood pressure.

According to an embodiment, the wearable electronic device 201 may identify a wake-up event indicating that the user has woken up based on the sensing value. According to an embodiment, the wearable electronic device 201 may display, through the display 260 (e.g., the display 260 of FIG. 2), information 1420 indicating that the wake-up event has been identified.

According to an embodiment, based on identifying the wake-up event, the wearable electronic device 201 may display, through the display 260, guide information 1430 (e.g., "Measure blood sugar now") for measuring blood sugar data using the external device 202 (e.g., the external device 202 of FIG. 2).

According to an embodiment, the wearable electronic device 201 may display, through the display 260, information 1410 on a current time and information 1440 on a progress status of obtaining the calibration data. For example, the information 1440 on the progress status of obtaining the calibration data may include a status bar indicating that 10% has progressed out of 100%.

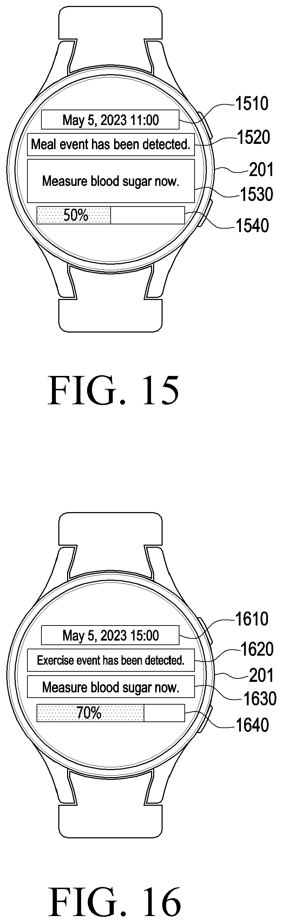

FIG. 15 is a view illustrating a screen displayed through a display when a wearable electronic device identifies a meal event according to an embodiment.

Referring to FIG. 15, according to an embodiment, the wearable electronic device 201 (e.g., the wearable electronic device 201 of FIG. 2) may obtain a sensing value through the at least one second sensor 212 (e.g., the at least one second sensor 212 of FIG. 2). For example, the sensing value may include a sensing value indicating at least one of a heart rate, an oxygen saturation, a stress, or a blood pressure.

According to an embodiment, the wearable electronic device 201 may identify a meal event indicating that the user is eating based on the sensing value. For example, the wearable electronic device 201 may identify the meal event by analyzing a sensing value indicating a movement of the user using an artificial intelligence model stored in the memory 210 (e.g., the memory 210 of FIG. 2). According to an embodiment, the wearable electronic device 201 may also identify the meal event based on a voice signal obtained using a microphone included in the wearable electronic device 201. In this case, the wearable electronic device 201 may also identify the meal event by further considering a voice signal obtained from an external electronic device wearable on an ear of the user.

According to an embodiment, the wearable electronic device 201 may display, through the display 260 (e.g., the display 260 of FIG. 2), information 1520 (e.g., "A meal event has been detected") indicating that the meal event has been identified.

According to an embodiment, the wearable electronic device 201 may determine a time point where the meal event is identified as a time point to measure blood sugar data of the user. According to an embodiment, the wearable electronic device 201 may also determine a time point where a specified first time elapses from a time point where the meal event is identified as a time point to measure blood sugar data of the user. The specified first time may include the time when a maximum blood sugar value is obtained. For example, the specified first time may include 1 hour.

According to an embodiment, the wearable electronic device 201 may display, through the display 260, guide information 1530 (e.g., "Measure blood sugar now") on a time point to measure blood sugar data of the user using the external device 202 (e.g., the external device 202 of FIG. 2).

According to an embodiment, the wearable electronic device 201 may display, through the display 260, information 1510 on a current time and information 1540 on a progress status of obtaining the calibration data. For example, the information 1540 on the progress status of obtaining the calibration data may include a status bar indicating that 50% has progressed out of 100%.

FIG. 16 is a view illustrating a screen displayed through a display when a wearable electronic device identifies an exercise event according to an embodiment.

Referring to FIG. 16, according to an embodiment, the wearable electronic device 201 (e.g., the wearable electronic device 201 of FIG. 2) may obtain a sensing value through the at least one second sensor 212 (e.g., the at least one second sensor 212 of FIG. 2). For example, the sensing value may include a sensing value indicating at least one of a heart rate, an oxygen saturation, a stress, or a blood pressure.

According to an embodiment, the wearable electronic device 201 may identify an exercise event indicating that the user is exercising based on the sensing value. According to an embodiment, the wearable electronic device 201 may display, through the display 260 (e.g., the display 260 of FIG. 2), information 1620 indicating that the exercise event has been identified.

According to an embodiment, the wearable electronic device 201 may determine a time point where the exercise event is identified as a time point to measure blood sugar data of the user. According to an embodiment, the wearable electronic device 201 may display, through the display 260, guide information 1630 (e.g., "Measure blood sugar now") on a time point to measure blood sugar data of the user using the external device 202 (e.g., the external device 202 of FIG. 2).

According to an embodiment, the wearable electronic device 201 may display, through the display 260, information 1610 on a current time and information 1640 on a progress status of obtaining the calibration data. For example, the information 1640 on the progress status of obtaining the calibration data may include a status bar indicating that 70% has progressed out of 100%.

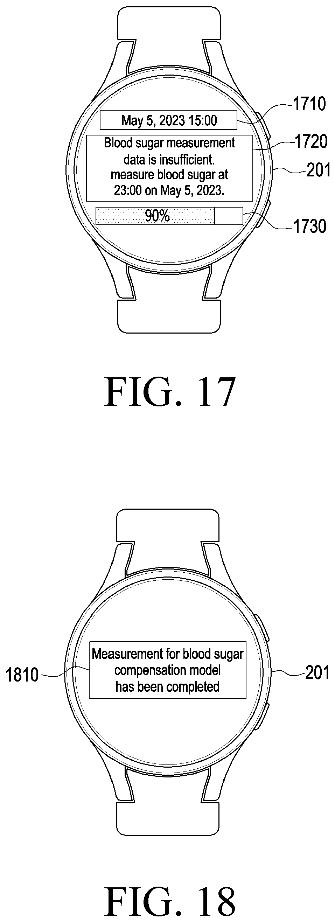

FIG. 17 is a view illustrating a screen displayed through a display when a wearable electronic device identifies that additional blood sugar data is needed according to an embodiment.

Referring to FIG. 17, according to an embodiment, the wearable electronic device 201 (e.g., the wearable electronic device 201 of FIG. 2) may determine a specific time point to obtain additional blood sugar data for obtaining the calibration data based on blood sugar values measured by the external device 202 (e.g., the external device 202 of FIG. 2).

According to an embodiment, the wearable electronic device 201 may display, through the display 260 (e.g., the display 260 of FIG. 2), information 1720 (e.g., "Blood sugar measurement data is insufficient. Measure blood sugar at 23:00 on May 5, 2023") on the specific time point to obtain additional blood sugar data.

According to an embodiment, the wearable electronic device 201 may display, through the display 260, information 1710 on a current time and information 1730 on a progress status of obtaining the calibration data. For example, the information 1730 on the progress status of obtaining the calibration data may include a status bar indicating that 90% has progressed out of 100%.

FIG. 18 is a view illustrating a screen displayed through a display when a wearable electronic device terminates a function of obtaining calibration data according to an embodiment.

Referring to FIG. 18, according to an embodiment, when the calibration data is obtained, the wearable electronic device 201 (e.g., the wearable electronic device 201 of FIG. 2) may display, through the display 260 (e.g., the display 260 of FIG. 2), information 1810 (e.g., "Measurement for blood sugar compensation model has been completed") indicating that the calibration data has been obtained.

According to an embodiment, the wearable electronic device 201 may also output information indicating that the calibration data has been obtained through auditory means or tactile means.

According to an embodiment, when the calibration data is obtained, the wearable electronic device 201 may terminate a function of obtaining the calibration data.

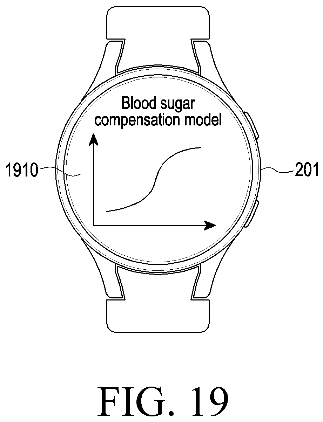

FIG. 19 is a view illustrating a screen on which a wearable electronic device displays calibration data according to an embodiment.

Referring to FIG. 19, according to an embodiment, the wearable electronic device 201 (e.g., the wearable electronic device 201 of FIG. 2) may display information 1910 on a calibration curve.

According to an embodiment, during an operation of obtaining the calibration data, the wearable electronic device 201 may also display information on blood sugar values obtained from the external device 202 (e.g., the external device 202 of FIG. 2).

According to an embodiment, the information on the blood sugar values obtained from the external device 202 may include the time when the blood sugar values are measured by the external device 202 and the blood sugar values (e.g., 120 mg/dl, 280 mg/dl, 260 mg/dl, 190 mg/dl).

According to an embodiment, the wearable electronic device 201 may also display information on blood sugar values including biometric signal values obtained through the first sensor 211 (e.g., the first sensor 211 of FIG. 2) and the time when the biometric signal values are measured.

The technical problems to be achieved in the disclosure are not limited to the technical problems mentioned above, and other technical problems not mentioned is clearly understood by those of ordinary skill in the art to which the disclosure belongs. According to an embodiment, the wearable electronic device 201 may include a first sensor 211, at least one second sensor 212, memory 210, a display 260, communication circuitry 290, and at least one processor 220.

According to an embodiment, the memory 210 may store instructions that, when executed by the at least one processor 220, cause the wearable electronic device 201 to identify a first time point to measure first blood sugar data of a user wearing the wearable electronic device 201 based on a first sensing value identified through the at least one second sensor 212.

According to an embodiment, the memory 210 may store instructions that, when executed by the at least one processor 220, cause the wearable electronic device 201 to display, through the display 260, first guide information for measuring a blood sugar of the user at the first time point using the external device 202.

According to an embodiment, the memory 210 may store instructions that, when executed by the at least one processor 220, cause the wearable electronic device 201 to measure, through the first sensor 211, a second sensing value indicating a first biometric signal of the user at the first time point.

According to an embodiment, the memory 210 may store instructions that, when executed by the at least one processor 220, cause the wearable electronic device 201 to obtain, through the communication circuitry 290, information indicating a first blood sugar value of the user measured by the external device 202 at the first time point.

According to an embodiment, the memory 210 may store instructions that, when executed by the at least one processor 220, cause the wearable electronic device 201 to obtain the calibration data for determining a blood sugar value of the user based on a sensing value indicating a biometric signal of the user to be measured using the first sensor 211, based on the second sensing value indicating the first biometric signal and the first blood sugar value.

According to an embodiment, the memory 210 may store instructions that, when executed by the at least one processor 220, cause the wearable electronic device 201 to identify a second time point different from the first time point to measure second blood sugar data of the user for obtaining the calibration data based on a third sensing value identified through the at least one second sensor 212. According to an embodiment, the memory 210 may store instructions that, when executed by the at least one processor, cause the wearable electronic device 201 to display, through the display 260, second guide information for measuring a blood sugar of the user using the external device 202 at the second time point.

According to an embodiment, the memory 210 may store instructions that, when executed by the at least one processor 220, cause the wearable electronic device 201 to measure, through the first sensor 211, a fourth sensing value indicating a second biometric signal of the user at the second time point.

According to an embodiment, the memory 210 may store instructions that, when executed by the at least one processor 220, cause the wearable electronic device 201 to obtain, through the communication circuitry 290, information indicating a second blood sugar value of the user measured by the external device 202 at the second time point.

According to an embodiment, the memory 210 may store instructions that, when executed by the at least one processor 220, cause the wearable electronic device 201 to obtain the calibration data based on the fourth sensing value indicating the second biometric signal and the second blood sugar value.

According to an embodiment, the memory 210 may store instructions that, when executed by the at least one processor 220, cause the wearable electronic device 201 to identify a third time point different from the first time point and the second time point to measure third blood sugar data of the user for obtaining the calibration data based on identifying that a difference between the first blood sugar value and the second blood sugar value is greater than a specified value.

According to an embodiment, the memory 210 may store instructions that, when executed by the at least one processor 220, cause the wearable electronic device 201 to display, through the display 260, third guide information for measuring a blood sugar of the user using the external device 202 at the third time point.

According to an embodiment, the memory 210 may store instructions that, when executed by the at least one processor 220, cause the wearable electronic device 201 to measure, through the first sensor 211, a first value after obtaining the calibration data.

According to an embodiment, the memory 210 may store instructions that, when executed by the at least one processor 220, cause the wearable electronic device 201 to identify a blood sugar value corresponding to the first value using the calibration data.

According to an embodiment, the memory 210 may store instructions that, when executed by the at least one processor 220, cause the wearable electronic device 201 to determine the blood sugar value corresponding to the first value as the blood sugar value of the user.

According to an embodiment, the memory 210 may store instructions that, when executed by the at least one processor 220, cause the wearable electronic device 201 to measure, through the first sensor 211, a second value.

According to an embodiment, the memory 210 may store instructions that, when executed by the at least one processor 220, cause the wearable electronic device 201 to update the calibration data based on identifying that a difference between the second value and the first value measured before the second value is greater than a specified value.

According to an embodiment, the memory 210 may store instructions that, when executed by the at least one processor 220, cause the wearable electronic device 201 to determine the first time point based on a time point where the meal event is identified when a meal event indicating that the user is eating is identified based on the first sensing value indicating at least one of a movement of the user or a heart rate of the user.

According to an embodiment, the memory 210 may store instructions that, when executed by the at least one processor 220, cause the wearable electronic device 201 to obtain, through the communication circuitry, blood sugar values measured for a specified time by the external device 202 that includes a continuous glucose monitoring system.

According to an embodiment, the memory 210 may store instructions that, when executed by the at least one processor 220, cause the wearable electronic device 201 to determine the first time point based on a time point where the meal event is identified when the meal event is identified based on identifying that a change of the blood sugar values during the specified time is greater than a specified value.

According to an embodiment, the memory 210 may store instructions that, when executed by the at least one processor 220, cause the wearable electronic device 201 to determine the first time point based on a time point where the exercise event is identified when an exercise event indicating that the user is exercising is identified based on the first sensing value indicating at least one of a movement of the user or a heart rate of the user.

According to an embodiment, the memory 210 may store instructions that, when executed by the at least one processor 220, cause the wearable electronic device 201 to determine the first time point based on a time point where the wake-up event is identified when a wake-up event indicating that the user has woken up is identified based on the first sensing value indicating at least one of a movement of the user or a heart rate of the user.

According to an embodiment, the calibration data may include a calibration curve indicating a relationship between at least one blood sugar value of the user measured by the external device 202 and a sensing value indicating at least one biometric signal of the user measured through the first sensor 211.

According to an embodiment, a method of operating a wearable electronic device 201 may include an operation of identifying a first time point to measure first blood sugar data of a user wearing the wearable electronic device to obtain calibration data based on a first sensing value identified through at least one second sensor 212 included in the wearable electronic device 201.

According to an embodiment, a method of operating a wearable electronic device 201 may include an operation of displaying, through a display 260 included in the wearable electronic device 201, first guide information for measuring a blood sugar of the user at the first time point using an external device 202.

According to an embodiment, a method of operating a wearable electronic device 201 may include an operation of measuring, through a first sensor 211 included in the wearable electronic device 201, a second sensing value indicating a first biometric signal of the user at the first time point.

According to an embodiment, a method of operating a wearable electronic device 201 may include an operation of obtaining, through communication circuitry 290 included in the wearable electronic device 201, information indicating a first blood sugar value of the user measured by the external device 202 at the first time point.

According to an embodiment, a method of operating a wearable electronic device 201 may include an operation of obtaining the calibration data for determining a blood sugar value of the user based on a sensing value indicating a biometric signal of the user to be measured using the first sensor 211, based on the first biometric signal value and the first blood sugar value.

According to an embodiment, a method of operating a wearable electronic device 201 may include an operation of identifying a second time point different from the first time point to measure second blood sugar data of the user for obtaining the calibration data based on a third sensing value identified through the at least one second sensor 212.

According to an embodiment, a method of operating a wearable electronic device 201 may include an operation of displaying, through the display 260, second guide information for measuring a blood sugar of the user using the external device 202 at the second time point.

According to an embodiment, a method of operating a wearable electronic device 201 may include an operation of measuring, through the first sensor 211, a fourth sensing value indicating a second biometric signal of the user at the second time point.

According to an embodiment, a method of operating a wearable electronic device 201 may include an operation of obtaining, through the communication circuitry 290, information indicating a second blood sugar value of the user measured by the external device 202 at the second time point.

According to an embodiment, a method of operating a wearable electronic device 201 may include an operation of obtaining the calibration data based on the fourth sensing value indicating the second biometric signal and the second blood sugar value.

According to an embodiment, a method of operating a wearable electronic device 201 may include an operation of identifying a third time point different from the first time point and the second time point to measure third blood sugar data of the user for obtaining the calibration data based on identifying that a difference between the first blood sugar value and the second blood sugar value is greater than a specified value.

According to an embodiment, a method of operating a wearable electronic device 201 may include an operation of displaying, through the display 260, third guide information for measuring a blood sugar of the user using the external device 202 at the third time point.

According to an embodiment, a method of operating a wearable electronic device 201 may include an operation of measuring, through the first sensor 211, a first value after obtaining the calibration data.

According to an embodiment, a method of operating a wearable electronic device 201 may include an operation of identifying a blood sugar value corresponding to the first value using the calibration data.

According to an embodiment, a method of operating a wearable electronic device 201 may include an operation of determining the blood sugar value corresponding to the first value as the blood sugar value of the user.

According to an embodiment, a method of operating a wearable electronic device 201 may include an operation of measuring, through the first sensor, a second value.

According to an embodiment, a method of operating a wearable electronic device 201 may include an operation of updating the calibration data based on identifying that a difference between the second value and the first value measured before the second value is greater than a specified value.

According to an embodiment, a method of operating a wearable electronic device 201 may include an operation of determining the first time point based on a time point where the meal event is identified when a meal event indicating that the user is eating is identified based on the first sensing value indicating at least one of a movement of the user or a heart rate of the user.

According to an embodiment, a method of operating a wearable electronic device 201 may include an operation of obtaining, through the communication circuitry, blood sugar values measured for a specified time by the external device 202.

According to an embodiment, a method of operating a wearable electronic device 201 may include an operation of determining the first time point based on a time point where the meal event is identified when the meal event is identified based on identifying that a change of the blood sugar values during the specified time is greater than a specified value.

According to an embodiment, a method of operating a wearable electronic device 201 may include an operation of determining the first time point based on a time point where the exercise event is identified when an exercise event indicating that the user is exercising is identified based on the first sensing value indicating at least one of a movement of the user or a heart rate of the user.

According to an embodiment, a method of operating a wearable electronic device 201 may include an operation of determining the first time point based on a time point where the wake-up event is identified when a wake-up event indicating that the user has woken up is identified based on the first sensing value indicating at least one of a movement of the user or a heart rate of the user.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of identifying a first time point to measure first blood sugar data of a user wearing the wearable electronic device to obtain calibration data based on a first sensing value identified through at least one second sensor 212 included in the wearable electronic device 201.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of displaying, through a display 260 included in the wearable electronic device 201, first guide information for measuring a blood sugar of the user at the first time point using an external device.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of measuring, through a first sensor included in the wearable electronic device, a second sensing value indicating a first biometric signal of the user at the first time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of obtaining, through communication circuitry 290 included in the wearable electronic device 201, information indicating a first blood sugar value of the user measured by the external device 202 at the first time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of obtaining the calibration data for determining a blood sugar value of the user based on a sensing value indicating a biometric signal of the user to be measured using the first sensor 211, based on the second sensing value indicating the first biometric signal and the first blood sugar value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of identifying a second time point different from the first time point to measure second blood sugar data of the user for obtaining the calibration data based on a third sensing value identified through the at least one second sensor 212.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of displaying, through the display 260, second guide information for measuring a blood sugar of the user using the external device 202 at the second time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of measuring, through the first sensor 211, a fourth sensing value indicating a second biometric signal of the user at the second time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of obtaining, through the communication circuitry 290, information indicating a second blood sugar value of the user measured by the external device 202 at the second time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of obtaining the calibration data based on the fourth sensing value indicating the second biometric signal and the second blood sugar value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of identifying a third time point different from the first time point and the second time point to measure third blood sugar data of the user for obtaining the calibration data based on identifying that a difference between the first blood sugar value and the second blood sugar value is greater than a specified value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of displaying, through the display 260, third guide information for measuring a blood sugar of the user using the external device 202 at the third time point.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of measuring, through the first sensor 211, a first value after obtaining the calibration data.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of identifying a blood sugar value corresponding to the first value using the calibration data.

According to an embodiment, in a storage medium storing computer-readable instructions, the instructions, when executed by at least one processor of a wearable electronic device 201, cause the wearable electronic device 201 to perform at least one operation. The at least one operation may include an operation of determining the blood sugar value corresponding to the first value as the blood sugar value of the user.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of measuring, through the first sensor 211, a second value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of updating the calibration data based on identifying that a difference between the second value and the first value measured before the second value is greater than a specified value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of determining the first time point based on a time point where the meal event is identified when a meal event indicating that the user is eating is identified based on the first sensing value indicating at least one of a movement of the user or a heart rate of the user.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of obtaining, through the communication circuitry 290, blood sugar values measured for a specified time by the external device 220.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of determining the first time point based on a time point where the meal event is identified when the meal event is identified based on identifying that a change of the blood sugar values during the specified time is greater than a specified value.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of determining the first time point based on a time point where the exercise event is identified when an exercise event indicating that the user is exercising is identified based on the first sensing value indicating at least one of a movement of the user or a heart rate of the user.

According to an embodiment, in a storage medium storing computer-readable instructions, the instructions, when executed by at least one processor 220 of a wearable electronic device 201, cause the wearable electronic device 201 to perform at least one operation. The at least one operation may include an operation of determining the first time point based on a time point where the wake-up event is identified when a wake-up event indicating that the user has woken up is identified based on the first sensing value indicating at least one of a movement of the user or a heart rate of the user.

Effects that may be obtained by the disclosure are not limited to the above-mentioned effects, and other effects not mentioned is clearly understood by those skilled in the art of the disclosure.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic device is not limited to the above-listed embodiments.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101 or 201). For example, a processor (e.g., the processor 120 or 220) of the machine (e.g., the electronic device 101 or 201) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{TM}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device (201) comprising:
a first sensor (211);
at least one second sensor (212);
a display (260);
communication circuitry (290);
at least one processor (220); and
memory (210) storing instructions,
wherein the instructions when executed by the at least one processor, cause the wearable electronic device to:
based on a first sensing value identified through the at least one second sensor, identify a first time point to measure first blood sugar data of a user wearing the wearable electronic device,
display, through the display, first guide information for measuring a blood sugar of the user at the first time point using an external device (202),
measure, through the first sensor, a second sensing value indicating a first biometric signal of the user at the first time point,
obtain, through the communication circuitry, information indicating a first blood sugar value of the user measured by the external device at the first time point, and
based on the second sensing value indicating the first biometric signal and the first blood sugar value, obtain calibration data for determining a blood sugar value of the user corresponding to a sensing value indicating a biometric signal of the user to be measured using the first sensor.

2. The wearable electronic device of claim 1, wherein the instructions, when executed by the at least one processor, cause the wearable electronic device to:
based on a third sensing value identified through the at least one second sensor, identify a second time point different from the first time point to measure second blood sugar data of the user,
display, through the display, second guide information for measuring a blood sugar of the user using the external device at the second time point,
measure, through the first sensor, a fourth sensing value indicating a second biometric signal of the user at the second time point,
obtain, through the communication circuitry, information indicating a second blood sugar value of the user measured by the external device at the second time point, and
based on the fourth sensing value indicating the second biometric signal and the second blood sugar value, obtain the calibration data.

3. The wearable electronic device of claim 1 or 2, wherein the instructions, when executed by the at least one processor, cause the wearable electronic device to:
based on identifying that a difference between the first blood sugar value and the second blood sugar value is greater than a specified value, identify a third time point different from the first time point and the second time point to measure third blood sugar data of the user to obtain the calibration data, and
display, through the display, third guide information for measuring a blood sugar of the user using the external device at the third time point.

4. The wearable electronic device of any one of claims 1 to 3, wherein the instructions, when executed by the at least one processor, cause the wearable electronic device to:
after obtaining the calibration data, measure, through the first sensor, a first value,
identify a blood sugar value corresponding to the first value using the calibration data, and
determine the blood sugar value corresponding to the first value as the blood sugar value of the user.

5. The wearable electronic device of any one of claims 1 to 4, wherein the instructions, when executed by the at least one processor, cause the wearable electronic device to:
measure, through the first sensor, a second value, and
based on identifying that a difference between the second value and the first value measured before the second value is greater than a specified value, update the calibration data.

6. The wearable electronic device of any one of claims 1 to 5, wherein the instructions, when executed by the at least one processor, cause the wearable electronic device to:
when a meal event indicating that the user is eating is identified based on the first sensing value indicating at least one of a movement of the user or a heart rate of the user, determine the first time point based on a time point at which the meal event is identified.

7. The wearable electronic device of any one of claims 1 to 6, wherein the instructions, when executed by the at least one processor, cause the wearable electronic device to:
obtain, through the communication circuitry, blood sugar values measured for a specified time by the external device that includes a continuous glucose monitoring system , and
when the meal event is identified based on identifying that a change of the blood sugar values during the specified time is greater than a specified value, determine the first time point based on a time point at which the meal event is identified.

8. The wearable electronic device of any one of claims 1 to 7, wherein the instructions, when executed by the at least one processor, cause the wearable electronic device to:
when an exercise event indicating that the user is exercising is identified based on the first sensing value indicating at least one of a movement of the user or a heart rate of the user, determine the first time point based on a time point at which the exercise event is identified.

9. The wearable electronic device of any one of claims 1 to 8, wherein the instructions, when executed by the at least one processor, cause the wearable electronic device to:
when a wake-up event indicating that the user has woken up is identified based on the first sensing value indicating at least one of a movement of the user or a heart rate of the user, determine the first time point based on a time point at which the wake-up event is identified.

10. The wearable electronic device of any one of claims 1 to 9, wherein the calibration data includes a calibration curve indicating a relationship between at least one blood sugar value of the user measured by the external device and at least one sensing value indicating at least one biometric signal of the user measured through the first sensor.

11. A method of operating a wearable electronic device (201), the method comprising:
based on a first sensing value identified through at least one second sensor (212) included in the wearable electronic device, identifying a first time point to measure first blood sugar data of a user wearing the wearable electronic device;
displaying, through a display (260) included in the wearable electronic device, first guide information for measuring a blood sugar of the user at the first time point using an external device (202);
measuring, through a first sensor (211) included in the wearable electronic device, a second sensing value indicating a first biometric signal of the user at the first time point;
obtaining, through communication circuitry (290) included in the wearable electronic device, information indicating a first blood sugar value of the user measured by the external device at the first time point; and
based on the second sensing value indicating the first biometric signal and the first blood sugar value, obtaining calibration data for determining a blood sugar value of the user corresponding to a sensing value indicating a biometric signal of the user to be measured using the first sensor.

12. The method of claim 11, further comprising:
based on a third sensing value identified through the at least one second sensor, identifying a second time point different from the first time point to measure second blood sugar data of the user to obtain the calibration data;
displaying, through the display, second guide information for measuring a blood sugar of the user using the external device at the second time point;
measuring, through the first sensor, a fourth sensing value indicating a second biometric signal of the user at the second time point;
obtaining, through the communication circuitry, information indicating a second blood sugar value of the user measured by the external device at the second time point; and
based on the fourth sensing value indicating the second biometric signal and the second blood sugar value, obtaining the calibration data.

13. The method of claim 11 or 12, further comprising:
based on identifying that a difference between the first blood sugar value and the second blood sugar value is greater than a specified value, identifying a third time point different from the first time point and the second time point to measure third blood sugar data of the user to obtain the calibration data; and
displaying, through the display, third guide information for measuring a blood sugar of the user using the external device at the third time point.

14. The method of any one of claims 11 to 13, further comprising:
after obtaining the calibration data, measuring, through the first sensor, a first value;
identifying a blood sugar value corresponding to the first value using the calibration data; and
determining the blood sugar value corresponding to the first value as the blood sugar value of the user.

15. A storage medium storing computer-readable instructions, wherein the instructions, when executed by at least one processor (220) of a wearable electronic device (201), cause the wearable electronic device to perform at least one operation, wherein the at least one operation includes:
based on a first sensing value identified through at least one second sensor (212) included in the wearable electronic device, identifying a first time point to measure first blood sugar data of a user wearing the wearable electronic device;
displaying, through a display (260) included in the wearable electronic device, first guide information for measuring a blood sugar of the user at the first time point using an external device;
measuring, through a first sensor (211) included in the wearable electronic device, a second sensing value indicating a first biometric signal of the user;
obtaining, through communication circuitry (290) included in the wearable electronic device, information indicating a first blood sugar value of the user measured by the external device at the first time point; and
based on the second sensing value indicating the first biometric signal and the first blood sugar value, obtaining calibration data for determining a blood sugar value of the user corresponding to a sensing value indicating a biometric signal of the user to be measured using the first sensor.
